# EUROPEAN PATENT SPECIFICATION

(11) **EP 3 866 692 B1**
(45) Date of publication and mention of the grant of the patent: **23.07.2025**
(21) Application number: 19872408.0
(22) Date of filing: 14.10.2019
(51) Int. Cl.: A61K 51/04

(54) **RADIOLIGANDS FOR IMAGING THE LPA1 RECEPTOR**
RADIOLIGANDEN ZUR ABBILDUNG DES LPA1-REZEPTORS
RADIOLIGANDS POUR L'IMAGERIE DU RÉCEPTEUR LPA1

(30) Priority: 15.10.2018 US 201862745524 P
(43) Date of publication of application: 25.08.2021
(73) Proprietor: Bristol-Myers Squibb Company, Princeton, NJ 08543 (US)
(72) Inventor: CHENG, Peter Tai Wah, Princeton, New Jersey 08543 (US); CORTE, James R., Princeton, New Jersey 08543 (US); DONNELLY, David J., Princeton, New Jersey 08543 (US); KIM, Joonyoung, Princeton, New Jersey 08543 (US); SHI, Jun, San Diego, CA 92130 (US); TAO, Shiwei, Princeton, New Jersey 08543 (US); TRAN, Tritin, King of Prussia, PA 19406 (US)
(74) Representative: Dehns
(86) International application number: PCT/US2019/056033
(87) International publication number: WO 2020/081410

(56) References cited:
- WO-A1-2017/210335
- WO-A1-2019/126094
- US-A1- 2016 152 735
- US-A1- 2016 256 577
- US-A1- 2017 360 759
- US-A1- 2019 185 446

## Description

### CROSS-REFERENCE TO RELATED APPLICATIONS

This application claims the priority benefit of U.S. Provisional Application No. 62/745,524, filed October 15, 2018.

### FIELD OF THE INVENTION

The invention relates to novel radiolabeled lysophosphatidic acid (LPA) receptor 1 antagonists and their use in labeling and diagnostic imaging of LPA1 receptors in mammals.

### BACKGROUND OF THE INVENTION

Positron emission tomography (PET) is a non-invasive imaging technique that can provide functional information about biological processes in living subjects. The ability to image and monitor *in* vivo molecular events, are great value to gain insight into biochemical and physiological processes in living organisms. This in turn is essential for the development of novel approaches for the treatment of diseases, early detection of disease and for the design of new drugs. PET relies on the design and synthesis of molecules labeled with positron-emitting radioisotope. These molecules are known as radiotracers or radioligands. For PET imaging, the most commonly used positron emitting (PET) radionuclides are; ¹¹C, ¹⁸F, ¹⁵O and ¹³N, all of which are cyclotron produced, and have half lives of 20, 110, 2 and 10 minutes, respectively. After being radiolabeled with a positron emitting radionuclide, these PET radioligands are administered to mammals, typically by intravenous (i.v.) injection. Once inside the body, as the radioligand decays it emits a positron that travels a small distance until it combines with an electron. An event known as an annihilation event then occurs, which generates two collinear photons with an energy of 511 keV each. Using a PET imaging scanner which is capable of detecting the gamma radiation emitted from the radioligand, planar and tomographic images reveal distribution of the radiotracer as a function of time. PET radioligands provide useful *in-vivo* information around target engagement and dose dependent receptor occupancy for human receptors.

Idiopathic pulmonary fibrosis (IPF) is a chronic disease that is characterized by the presence of scar tissue within the lungs, breathlessness, and chronic dry cough. IPF belongs to a family of lung disorders known as interstitial lung disease (ILD) and is associated with the pathological pattern known as usual interstitial pulmonary fibrosis (UIP). There are several potential clinical courses for IPF including slowly progressive disease (most common), disease marked by episodic acute exacerbations, or rapidly progressive disease. The median survival time from the time of diagnosis is between 2 and 5 years. There is currently no cure for IPF, and the available treatment options are very limited and have undesirable side effects. The pathogenesis of IPF is unknown but one of the hypotheses is that an initial injury to epithelial cells increases lysophosphatidic acid (LPA) production. LPA is a bioactive phospholipid that regulates numerous aspects of cellular function and has been recognized as a novel mediator of wound healing and tissue fibrosis. LPA mediates its biological effects through the LPA receptors, of which at least six isoforms have been identified. Recent studies have recently linked the LPA1 isoform to the pathogenesis of lung fibrosis and the LPA1 receptor has been identified as a potential clinical target for IPF. Several findings support the role of LPA/LPA1 pathway in IPF which activates LPA1 receptors, leading to endothelial barrier breakdown, inflammation, and fibroblast recruitment/proliferation. LPA is elevated in bronchoalveolar lavage (BAL) of IPF patients. LPA concentrations are increased in BAL fluid (BALF) in persons with IPF and LPA1 antagonism inhibits fibroblast migration induced by IPF BALF. Also, knockout mice lacking the LPA1 receptor show reduced vascular leakage and decreased collagen accumulation in the lungs in a bleomycin model of fibrosis. Based on these data, LPA1 signaling is thought to contribute to the development of lung fibrosis, at least in part, through the induction of vascular leakage and stimulation of fibroblast migration.

Use of a specific PET radioligand having high affinity for the LPA1 receptor in conjunction with supporting imaging technology may provide a method for clinical evolution around both target engagement and dose/occupancy relationships of LPA1 antagonists in the human lung LPA1 or LPA1 in other organs such as the kidneys, liver, heart or skin. The invention described herein relates to radiolabeled LPA1 antagonists that would be useful for the exploratory and diagnostic imaging applications, both in-vitro and in-vivo, and for competition studies using radiolabeled and unlabeled LPA1 antagonists.

U.S. Patent Application Publication No. 2017/0360759 (PCT Application Publication No. WO2017/223016) discloses certain antagonists of lysophosphatic acid receptors for use in treating LPA-dependent or LPA-mediated conditions or diseases such as fibrosis of various organs, including the lung. U.S. Patent Application Publication No. 2016/256577 discloses in particular 11C-labelled compounds for PET.

### SUMMARY

The present disclosure is based, in part, on the appreciation that radiolabeled lysophosphatidic acid (hereinafter "LPA1") receptor antagonists are useful in the detection and/or quantification and/or imaging of LPA1 receptors and/or LPA1 expression and/or affinity of a compound for occupying LPA1 receptors, in tissue of a mammalian species. It has been found that radiolabeled LPA1 receptor antagonists, when administered to a mammalian species, build up at or occupy LPA1 receptors and can be detected through imaging techniques, thereby providing valuable diagnostic markers for presence of LPA1 receptors, affinity of a compound for occupying LPA1 receptors, and clinical evaluation and dose selection of LPA1 receptor antagonists. In addition, the radiolabeled LPA1 receptor antagonists disclosed herein can be used as a research tool to study the interaction of unlabeled LPA1 receptor antagonists with LPA1 receptors *in vivo* via competition between the unlabeled drug and the radiolabeled drug for binding to the receptor. These types of studies are useful in determining the relationship between LPA 1 receptor occupancy and dose of unlabeled LPA1 receptor antagonist, as well as for studying the duration of blockade of the receptor by various doses of unlabeled LPA 1 receptor antagonists.

As a clinical tool, the radiolabeled LPA1 receptor antagonist can be used to help define clinically efficacious doses of LPA1 receptor antagonists. In animal experiments, the radiolabeled LPA1 receptor antagonist can be used to provide information that is useful for choosing between potential drug candidates for selection for clinical development. The radiolabeled LPA1 receptor antagonist can also be used to study the regional distribution and concentration of LPA1 receptors in living lung tissue and other tissue, such as kidney, heart, liver and skin, of humans and animals and in tissue samples. They can be used to study disease or pharmacologically related changes in LPA1 receptor concentrations.

The present disclosure provides a radiolabeled compound of Formula (I): 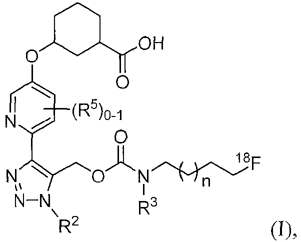 or a pharmaceutically acceptable salt thereof, wherein:
R² and R³ are independently C₁₋₄ alkyl;
R⁵ is independently F, Cl, C₁₋₄ alkyl or C₁₋₄ haloalkyl; and
n is independently 0, 1, 2 or 3.

In one aspect of the present disclosure, the following radiolabeled compound of Formula (Ia) is provided: 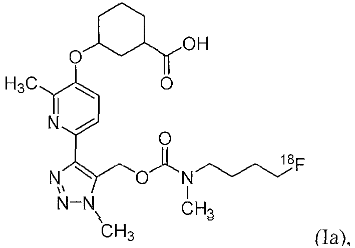 or a pharmaceutically acceptable salt thereof.

In the present invention, the following radiolabeled compound of Formula (IIa) is provided: 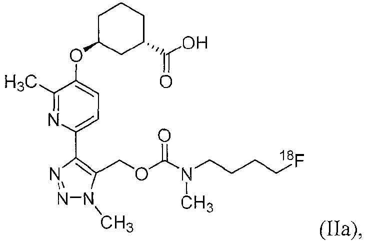 or a pharmaceutically acceptable salt thereof.

According to one embodiment of the present invention, pharmaceutical and diagnostic compositions are provided. Such pharmaceutical or diagnostic composition comprises a radiolabeled compound of Formula (IIa), or a pharmaceutically acceptable salt thereof; and a pharmaceutically acceptable carrier therefor. In one embodiment, the radiolabeled compound of Formula (IIa), or a pharmaceutically acceptable salt thereof is present in a therapeutically effective amount and diagnostically effect amount in the pharmaceutical and diagnostic compositions, respectively.

In one embodiment, the present invention provides a method of *in vivo* imaging of mammalian tissues of known LPA1 expression comprising the steps of:
(a) administering the radiolabeled compound of Formula (IIa), or a pharmaceutically acceptable salt thereof, to a mammalian species; and
(b) imaging *in vivo* the distribution of the radiolabeled compound by positron emission tomography (PET) scanning.

In another embodiment, the present invention provides a method for screening a non-radiolabeled compound to determine its affinity for occupying the binding sites of LPA1 receptors in mammalian tissue comprising the steps of:
(a) administering the radiolabeled compound of Formula (IIa), or a pharmaceutically acceptable salt thereof, to a mammalian species;
(b) imaging *in vivo* tissues of known LPA1 expression by positron emission tomography (PET) to determine a baseline uptake of the radiolabeled compound;
(c) administering the non-radiolabeled compound, or a pharmaceutically acceptable salt thereof, to the mammalian species;
(d) administering a second dose of the radiolabeled compound of Formula (IIa), or a pharmaceutically acceptable salt thereof, to the mammalian species;
(e) imaging *in vivo* the distribution of the radiolabeled compound of (IIa) in tissues that express LPA1 receptors;
(f) comparing the signal from PET scan data at the baseline within the tissue that expresses LPA1 to PET scan data retrieved after administering the non-radiolabeled compound within the tissue that expresses LPA1 receptors.

In another embodiment, the present invention provides a method for monitoring the treatment of a mammalian patient who is being treated with an LPA1 receptor antagonist comprising the steps of:
(a) administering to the patient the radiolabeled compound of Formula (IIa), or a pharmaceutically acceptable salt thereof;
(b) obtaining an image of tissues in the patient that express LPA1 receptors by positron emission tomography (PET); and
(c) detecting to what degree the radiolabeled compound occupies the binding site of the LPA1 receptor.

In another embodiment, the present invention provides a method for tissue imaging comprising the steps of contacting a tissue that contains LPA1 receptors with the radiolabeled compound of Formula (IIa), or a pharmaceutically acceptable salt thereof; and detecting the radiolabeled compound using positron emission tomography (PET) imaging. In such a method, the radiolabeled compound can be detected either *in vitro* or *in vivo.*

In another embodiment, the present invention provides the compound of formula (IIa) for use in a method for diagnosing the presence of a fibrotic disease in a mammalian species, comprising the steps of
(a) administering to a mammalian species in need thereof the radiolabeled compound of Formula (IIa), or a pharmaceutically acceptable salt thereof, which binds to the LPA1 receptor associated with the presence of the fibrotic disease; and
(b) obtaining a radio-image of at least a portion of the mammalian species to detect the presence or absence of the radiolabeled compound; wherein the presence and location of the radiolabeled compound above background is indicative of the presence or absence of the fibrotic disease.

In another embodiment, the present invention provides the compound of formula (IIa) for use in a method for diagnosing the presence of a fibrotic disease, for example, idiopathic pulmonary fibrosis, in a mammalian species, comprising the steps of
(a) administering to a mammalian species in need thereof the radiolabeled compound of Formula (IIa), or a pharmaceutically acceptable salt thereof, which binds to the LPA1 receptor associated with the presence of the fibrotic disease;
(b) detecting radioactive emission of the radiolabeled compound for the mammalian species;
(c) comparing the radioactive emission from the radiolabeled compound for the mammalian species with standard values; and
(d) finding any significant deviation between the radioactive emission detected for the mammalian species as compared with standard values, and attributing the deviation to the fibrotic disease.

In another embodiment, the present invention provides a method for quantifying diseased cells or tissues in a mammalian species, comprising the steps of
(a) administering to a mammalian species having diseased cells or tissues the radiolabeled compound of Formula (IIa), or a pharmaceutically acceptable salt thereof, which binds to LPA1 receptors located within the diseased cells or tissues; and
(b) detecting radioactive emissions of the radiolabeled compound in the diseased cells or tissues, wherein the level and distribution of the radioactive emissions in the diseased cells or tissues are a quantitative measure of the diseased cells or tissues.

In another aspect, the present disclosure provides a method for separating a preferred diastereomer of Formula (IIa) from a mixture with the diastereomer of Formula (IIc) using potassium carbonate, 18F-fluoride and a phase transfer catalyst, e.g., kyptofix 2.2.2., followed by saponification reaction in sodium hydroxide.

### BRIEF DESCRIPTION OF THE DRAWINGS

FIG. 1 depicts the semi-preparative HPLC chromatogram of ¹⁸F-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid.
FIG. 2 depicts the analytical HPLC chromatogram of ¹⁸F-(1S,3 S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid.
FIG. 3 depicts the analytical chiral HPLC chromatogram of ¹⁸F-(1S,3 S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid co-injected with reference standard.
FIG. 4 depicts the whole cell binding of ¹⁸F-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid to CHO cells heterologously expressing human LPA1.
FIG. 5 depicts the representative PET and transmission scan co-registered summed images 60-90 minutes post injection of ¹⁸F-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in wild type and bleomycin treated rats.
FIG. 6 depicts a graphical representation of the percent displacement of ¹⁸F-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in rat lung as function of pre-dosing multiples doses of a LPA1 antagonist.
FIG. 7 depicts the representative PET/MRI Images of ¹⁸F-(1S,3 S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in cynomolgus monkey at baseline and after administration of vehicle or LPA1 antagonist at 3, 10, and 30 mg/kg.
FIG. 8 depicts the graphical representation of the percent displacement of ¹⁸F-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in cynomolgus monkey lung tissues after treatment with a LPA1 antagonist or vehicle.

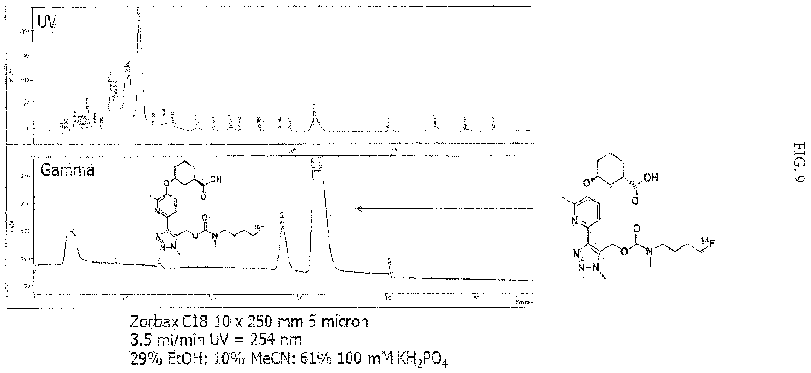

FIG. 9 depicts the semi-preparative HPLC chromatogram of ¹⁸F-(1R,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid.

### DETAILED DESCRIPTION OF THE INVENTION

The present disclosure provides a radiolabeled compound of Formula (I): 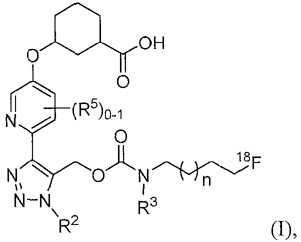 or a pharmaceutically acceptable salt thereof, wherein:
R² and R³ are independently C₁₋₄ alkyl;
R⁵ is independently F, Cl, C₁₋₄ alkyl or C₁₋₄ haloalkyl; and
n is independently 0, 1, 2 or 3.

In one aspect, the present disclosure provides a radiolabeled compound of Formula (Ia): 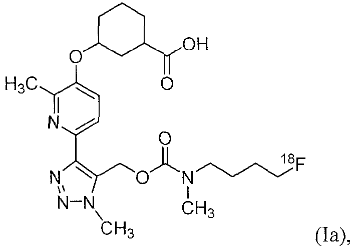 or a pharmaceutically acceptable salt thereof.

Stereoisomers of Formula (Ia) are for example, the following:

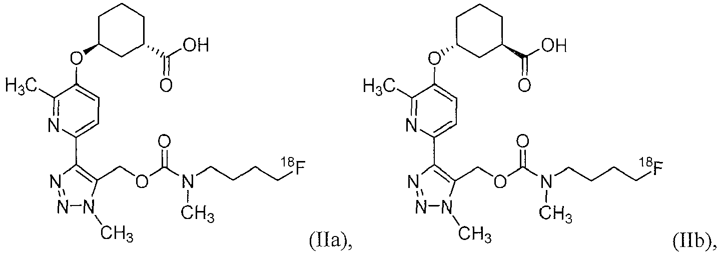

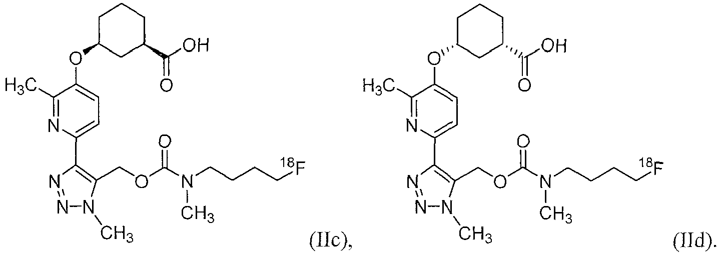

The compound of Formula (I), (Ia) or (IIa) is a radiolabeled LPA1 receptor antagonist which is useful as a positron emitting molecule having LPA1 receptor affinity. The term "radiolabeled LPA1 receptor antagonist" as used herein refers to a compound of Formula (I), (Ia) or (IIa), or a pharmaceutically acceptable salt thereof.

In another embodiment, the present disclosure provides a diagnostic composition for imaging LPA1 receptors which includes a radiolabeled LPA1 receptor antagonist, i.e., a compound of Formula (IIa), or a pharmaceutically acceptable salt thereof, and a pharmaceutically acceptable carrier therefor. In still another embodiment, the present disclosure provides a pharmaceutical composition which includes a radiolabeled LPA1 receptor antagonist, i.e., a compound of Formula (IIa) or a pharmaceutically acceptable salt thereof, and a pharmaceutically acceptable carrier therefor. In yet another embodiment, the present disclosure provides a method of autoradiography of mammalian tissues of known LPA1 expression, which includes the steps of administering a radiolabeled LPA1 receptor antagonist to a mammalian species, obtaining an image of the tissues by positron emission tomography, and detecting the radiolabeled compound in the tissues to determine LPA1 target engagement and LPA1 receptor occupancy of said tissues.

Radiolabeled LPA1 receptor antagonists, when labeled with the appropriate radionuclide, are potentially useful for a variety of *in vitro* and/or *in vivo* imaging applications, including diagnostic imaging, basic research, and radiotherapeutic applications. Specific examples of possible diagnostic imaging and radiotherapeutic applications include determining the location of, the relative activity of and/or quantification of LPA1 receptors; radioimmunoassay of LPA 1 receptor antagonist; and autoradiography to determine the distribution of LPA1 receptors in a mammal or an organ or tissue sample thereof.

In particular, the instant radiolabeled LPA1 receptor antagonists are useful for positron emission tomographic (PET) imaging of LPA1 receptors in the lung, heart, kidneys, liver and skin and other organs of living humans and experimental animals. These radiolabeled LPA1 receptor antagonists may be used as research tools to study the interaction of unlabeled LPA1 receptor antagonists with LPA1 receptors *in vivo* via competition between the unlabeled drug and the radiolabeled compound for binding to the receptor. These types of studies are useful for determining the relationship between LPA1 receptor occupancy and dose of unlabeled LPA1 receptor antagonist, as well as for studying the duration of blockade of the receptor by various doses of the unlabeled LPA1 receptor antagonist. As a clinical tool, the radiolabeled LPA1 receptor antagonists may be used to help define a clinically efficacious dose of an LPA1 receptor antagonist. In animal experiments, the radiolabeled LPA1 receptor antagonists can be used to provide information that is useful for choosing between potential drug candidates for selection for clinical development. The radiolabeled LPA1 receptor antagonists may also be used to study the regional distribution and concentration of LPA1 receptors in the human lung, kidney, liver, skin, heart, and other organs of living experimental animals and in tissue samples. The radiolabeled LPA1 receptor antagonists may also be used to study disease or pharmacologically related changes in LPA1 receptor concentrations.

For example, positron emission tomography (PET) tracers such as the present radiolabeled LPA1 receptor antagonists can be used with currently available PET technology to obtain the following information: relationship between level of receptor occupancy by candidate LPA1 receptor antagonists and clinical efficacy in patients; dose selection for clinical trials of LPA1 receptor antagonist prior to initiation of long term clinical studies; comparative potencies of structurally novel LPA1 receptor antagonists; investigating the influence of LPA1 receptor antagonists on *in vivo* transporter affinity and density during the treatment of clinical targets with LPA1 receptor antagonists; changes in the density and distribution of LPA1 receptors during effective and ineffective treatment of idiopathic pulmonary fibrosis, cardiac fibrosis, or other fibrotic diseases.

The present radiolabeled LPA1 receptor antagonists have utility in imaging LPA1 receptors or for diagnostic imaging with respect to a variety of disorders associated with LPA1 receptors.

The terms "fibrosis" or "fibrotic disease", as used herein, refers to conditions that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract, such as idiopathic pulmonary fibrosis, scleroderma, and chronic nephropathies.

Exemplary diseases, disorders, or conditions that involve fibrosis include, but are not limited to: lung diseases associated with fibrosis, *e.g*., idiopathic pulmonary fibrosis, pulmonary fibrosis secondary to systemic inflammatory disease such as rheumatoid arthritis, scleroderma, lupus, cryptogenic fibrosing alveolitis, radiation induced fibrosis, chronic obstructive pulmonary disease (COPD), chronic asthma, silicosis, asbestos induced pulmonary or pleural fibrosis, acute lung injury and acute respiratory distress (including bacterial pneumonia induced, trauma induced, viral pneumonia induced, ventilator induced, non-pulmonary sepsis induced, and aspiration induced); chronic nephropathies associated with injury/fibrosis (kidney fibrosis), *e.g.,* glomerulonephritis secondary to systemic inflammatory diseases such as lupus and scleroderma, diabetes, glomerular nephritis, focal segmental glomerular sclerosis, IgA nephropathy, hypertension, allograft and Alport; gut fibrosis, *e.g.,* scleroderma, and radiation induced gut fibrosis; liver fibrosis, *e.g.,* cirrhosis, alcohol induced liver fibrosis, nonalcoholic steatohepatitis (NASH), biliary duct injury, primary biliary cirrhosis, infection or viral induced liver fibrosis (*e.g.,* chronic HCV infection), and autoimmune hepatitis; head and neck fibrosis, *e.g.,* radiation induced; corneal scarring, *e.g.,* LASIK (laser-assisted in situ keratomileusis), corneal transplant, and trabeculectomy; hypertrophic scarring and keloids, *e.g.,* burn induced or surgical; and other fibrotic diseases, *e.g.,* sarcoidosis, scleroderma, spinal cord injury/fibrosis, myelofibrosis, vascular restenosis, atherosclerosis, arteriosclerosis, Wegener's granulomatosis, mixed connective tissue disease, and Peyronie's disease.

Other diseases, disorders, or conditions where LPA1 receptors may be involved include atherosclerosis, thrombosis, heart disease, vasculitis, formation of scar tissue, restenosis, phlebitis, COPD (chronic obstructive pulmonary disease), pulmonary hypertension, pulmonary fibrosis, pulmonary inflammation, bowel adhesions, bladder fibrosis and cystitis, fibrosis of the nasal passages, sinusitis, inflammation mediated by neutrophils, and fibrosis mediated by fibroblasts, dermatological disorders including proliferative or inflammatory disorders of the skin such as, atopic dermatitis, bullous disorders, collagenosis, psoriasis, psoriatic lesions, dermatitis, contact dermatitis, eczema, rosacea, wound healing, scarring, hypertrophic scarring, keloids, Kawasaki Disease, rosacea, Sjögren-Larsson Syndrome, and urticaria, respiratory diseases including asthma, adult respiratory distress syndrome and allergic (extrinsic) asthma, non-allergic (intrinsic) asthma, acute severe asthma, chronic asthma, clinical asthma, nocturnal asthma, allergen-induced asthma, aspirin-sensitive asthma, exercise-induced asthma, isocapnic hyperventilation, child-onset asthma, adult-onset asthma, cough-variant asthma, occupational asthma, steroid-resistant asthma, seasonal asthma, seasonal allergic rhinitis, perennial allergic rhinitis, chronic obstructive pulmonary disease, including chronic bronchitis or emphysema, pulmonary hypertension, interstitial lung fibrosis and/or airway inflammation and cystic fibrosis, and hypoxia, and inflammatory/immune disorders including psoriasis, rheumatoid arthritis, vasculitis, inflammatory bowel disease, dermatitis, osteoarthritis, asthma, inflammatory muscle disease, allergic rhinitis, vaginitis, interstitial cystitis, scleroderma, eczema, allogeneic or xenogeneic transplantation (organ, bone marrow, stem cells and other cells and tissues) graft rejection, graft-versus-host disease, lupus erythematosus, inflammatory disease, type I diabetes, pulmonary fibrosis, dermatomyositis, Sjögren's syndrome, thyroiditis (*e.g.,* Hashimoto's and autoimmune thyroiditis), myasthenia gravis, autoimmune hemolytic anemia, multiple sclerosis, cystic fibrosis, chronic relapsing hepatitis, primary biliary cirrhosis, allergic conjunctivitis and atopic dermatitis.

For the use of the instant compounds as exploratory or diagnostic imaging agents, the radiolabeled compounds may be administered to mammals, preferably humans, in a pharmaceutical composition, either alone or, preferably, in combination with pharmaceutically acceptable carriers or diluents, optionally with known adjuvants, such as alum, in a pharmaceutical composition, according to standard pharmaceutical practice. Such compositions can be administered orally or parenterally, including the intravenous, intramuscular, intraperitoneal, subcutaneous, rectal and topical routes of administration. Preferably, administration is intravenous. The LPA1 receptor antagonists are radiotracers labeled with short-lived, positron emitting radionuclides and thus are generally administered via intravenous injection within less than one hour of their synthesis. This is necessary because of the short half-life of the radionuclides involved.

An appropriate dosage level for the unlabeled LPA1 receptor antagonist can range from 1 mg to 5000 mg per day and is preferably from 20 mg to 1000 mg per day. When the present radiolabeled LPA1 receptor antagonist is administered into a human subject, the amount required for imaging will normally be determined by the prescribing physician with the dosage generally varying according to the quantity of emission from the radionuclide. However, in most instances, an effective amount will be the amount of compound sufficient to produce emissions in the range of from about 1-10 mCi. In one exemplary application, administration occurs in an amount between 0.5-20 mCi of total radioactivity injected into a mammal depending upon the subjects body weight. The upper limit is set by the dosimetry of the radiolabeled molecule in either rodent or non-human primate.

The following illustrative procedure may be utilized when performing PET imaging studies on patients in the clinic. The patient is premedicated with unlabeled LPA1 receptor antagonist some time prior to the day of the experiment and is fasted for at least 12 hours allowing water intake ad libitum. A 20 G two-inch venous catheter is inserted into the contralateral ulnar vein for radiotracer administration. Administration of the PET tracer is often timed to coincide with time of maximum (Tₘₐₓ) or minimum (Tₘᵢₙ) of LPA1 receptor antagonist concentration in the blood.

The patient is positioned in the PET camera and a tracer dose of the PET tracer of radiolabeled LPA1 receptor antagonist such as [¹⁸F] Example 1 (<20 mCi) is administered via i.v. catheter. Either arterial or venous blood samples are taken at appropriate time intervals throughout the PET scan in order to analyze and quantitate the fraction of unmetabolized PET tracer of [¹⁸F] Example 1 in plasma. Images are acquired for up to 120 min. Within ten minutes of the injection of radiotracer and at the end of the imaging session, 1 ml blood samples are obtained for determining the plasma concentration of any unlabeled LPA1 receptor antagonist which may have been administered before the PET tracer.

Tomographic images are obtained through image reconstruction. For determining the distribution of radiotracer, regions of interest (ROIs) are drawn on the reconstructed image including, but not limited to, the lungs, liver, heart, kidney, skin or other organs and tissue. Radiotracer uptakes over time in these regions are used to generate time activity curves (TAC) obtained in the absence of any intervention or in the presence of the unlabeled LPA1 receptor antagonist at the various dosing paradigms examined. Data are expressed as radioactivity per unit time per unit volume (µCi/cc/mCi injected dose). TAC data are processed with various methods well-known in the field to yield quantitative parameters, such as Binding Potential (BP) or Volume of Distribution (V_{T}), that are proportional to the density of unoccupied LPA1 receptor. Inhibition of LPA1 receptor is then calculated based on the change of BP or V_{T} by equilibrium analysis in the presence of LPA1 receptor antagonists at the various dosing paradigms as compared to the BP or V_{T} in the unmedicated state. Inhibition curves are generated by plotting the above data vs the dose (concentration) of LPA1 receptor antagonists. Inhibition of LPA1 receptor is then calculated based on the maximal reduction of PET radioligand's V_{T} or BP that can be achieved by a blocking drug at Eₘₐₓ, Tₘₐₓ or Tₘᵢₙ and the change of its nonspecific volume of distribution (*V*_{ND}) and the BP in the presence of LPA1 receptor antagonists at the various dosing paradigms as compared to the BP or V_{T} in the unmedicated state. The ID50 values are obtained by curve fitting the dose-rate/inhibition curves.

The present disclosure is further directed to a method for the diagnostic imaging of LPA1 receptors in a mammal in need thereof which includes the step of combining radiolabeled LPA1 receptor antagonist with a pharmaceutical carrier or excipient.

### DEFINITIONS

Unless otherwise stated, the following terms used in this application, including the specification and claims, have the definitions given below. It must be noted that, as used in the specification and the appended claims, the singular forms "a", "an" and "the" include plural referents unless the context clearly dictates otherwise. Unless otherwise indicated, conventional methods of mass spectroscopy, NMR, HPLC, protein chemistry, biochemistry, recombinant DNA techniques and pharmacology are employed. Furthermore, use of the term "including" as well as other forms, such as "include", "includes", and "included", is not limiting. The section headings used herein are for organizational purposes only and are not to be construed as limiting the subject matter described.

The term "acceptable" with respect to a formulation, composition or ingredient, as used herein, means having no persistent detrimental effect on the general health of the subject being treated.

The term "modulate", as used herein, means to interact with a target either directly or indirectly so as to alter the activity of the target, including, by way of example only, to enhance the activity of the target, to inhibit the activity of the target, to limit the activity of the target, or to extend the activity of the target.

The term "modulator", as used herein, refers to a molecule that interacts with a target either directly or indirectly. The interactions include, but are not limited to, the interactions of an agonist, partial agonist, an inverse agonist and antagonist. In one embodiment, a modulator is an antagonist.

The term "agonist", as used herein, refers to a molecule such as a compound, a drug, an enzyme activator or a hormone modulator that binds to a specific receptor and triggers a response in the cell. An agonist mimics the action of an endogenous ligand (such as LPA, prostaglandin, hormone or neurotransmitter) that binds to the same receptor.

The term "antagonist", as used herein, refers to a molecule such as a compound, which diminishes, inhibits, or prevents the action of another molecule or the activity of a receptor site. Antagonists include, but are not limited to, competitive antagonists, non-competitive antagonists, uncompetitive antagonists, partial agonists and inverse agonists.

The term "LPA-dependent", as used herein, refers to conditions or disorders that would not occur, or would not occur to the same extent, in the absence of LPA.

The term "LPA-mediated", as used herein, refers to refers to conditions or disorders that might occur in the absence of LPA but can occur in the presence of LPA.

The terms "co-administration" or the like, as used herein, are meant to encompass administration of the selected therapeutic agents to a single patient, and are intended to include treatment regimens in which the agents are administered by the same or different route of administration or at the same or different time.

The term "composition" as used herein is intended to encompass a product comprising the specified ingredients in the specified amounts, as well as any product which results, directly or indirectly, from combination of the specified ingredients in the specified amounts. Such term in relation to pharmaceutical composition, is intended to encompass a product comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or ignore of the ingredient. Accordingly, the pharmaceutical compositions of the present invention encompass any composition made by mixing a compound of the present invention and a pharmaceutically acceptable carrier. By "pharmaceutically acceptable" it is meant the carrier, diluent or excipient must be compatible with the other ingredients of the formulation and not deleterious to the recipient thereof. The terms "administration of" and or "administering a" compound should be understood to mean providing a compound of the invention or a prodrug of a compound of the invention to the patient.

The terms "effective amount" or "therapeutically effective amount", as used herein, refer to a sufficient amount of an agent or a compound being administered which will relieve to some extent one or more of the symptoms of the disease or condition being treated. The result can be reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case may be determined using techniques, such as a dose escalation study.

The term "pharmaceutical combination" as used herein, means a product that results from the mixing or combining of more than one active ingredient and includes both fixed and non-fixed combinations of the active ingredients. The term "fixed combination" means that the active ingredients, *e.g.,* a compound of Formula (I), (Ia) or (IIa) and a co-agent, are both administered to a patient simultaneously in the form of a single entity or dosage. The term "non-fixed combination" means that the active ingredients, *e.g.,* a compound of Formula (I), (Ia) or (IIa) and a co-agent, are administered to a patient as separate entities either simultaneously, concurrently or sequentially with no specific intervening time limits, wherein such administration provides effective levels of the two compounds in the body of the patient. The latter also applies to cocktail therapy, *e.g.,* the administration of three or more active ingredients.

The term "subject" or "patient" encompasses mammals. Examples of mammals include, but are not limited to, humans, chimpanzees, apes, monkey, cattle, horses, sheep, goats, swine, rabbits, dogs, cats, rodents, rats, mice guinea pigs, and the like. In one embodiment, the mammal is a human.

The terms "treat", "treating" or "treatment", as used herein, include alleviating, abating or ameliorating at least one symptom of a disease or condition, preventing additional symptoms, inhibiting the disease or condition, *e.g.,* arresting the development of the disease or condition, relieving the disease or condition, causing regression of the disease or condition, relieving a condition caused by the disease or condition, or stopping the symptoms of the disease or condition either prophylactically and/or therapeutically.

The compounds herein described may have asymmetric centers. Such compounds containing an asymmetrically substituted atom may be isolated in optically active or racemic forms. It is well known in the art how to prepare optically active forms, such as by resolution of racemic forms or by synthesis from optically active starting materials. All chiral, diastereomeric, and racemic forms, of a structure are intended, unless the specific stereochemistry or isomeric form is specifically indicated.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof.

The terms pharmaceutically acceptable "salt" and "salts" may refer to basic salts formed with inorganic and organic bases. Such salts include ammonium salts; alkali metal salts, such as lithium, sodium, and potassium salts; alkaline earth metal salts, such as calcium and magnesium salts; salts with organic bases, such as amine like salts (*e.g.,* dicyclohexylamine salt, benzathine, N-methyl-D-glucamine, and hydrabamine salts); and salts with amino acids like arginine, lysine, and the like; and zwitterions, the so-called "inner salts". Nontoxic, pharmaceutically acceptable salts are preferred, although other salts are also useful, *e.g.,* in isolating or purifying the product.

The term pharmaceutically acceptable "salt" and "salts" also includes acid addition salts. These are formed, for example, with strong inorganic acids, such as mineral acids, for example sulfuric acid, phosphoric acid, or a hydrohalic acid such as HCl or HBr, with strong organic carboxylic acids, such as alkanecarboxylic acids of 1 to 4 carbon atoms which are unsubstituted or substituted, for example, by halogen, for example acetic acid, such as saturated or unsaturated dicarboxylic acids, for example oxalic, malonic, succinic, maleic, fumaric, phthalic, or terephthalic acid, such as hydroxycarboxylic acids, for example ascorbic, glycolic, lactic, malic, tartaric, or citric acid, such as amino acids, (for example aspartic or glutamic acid or lysine or arginine), or benzoic acid, or with organic sulfonic acids, such as (C₁-C₄) alkyl or arylsulfonic acids, which are unsubstituted or substituted, for example by halogen, for example methanesulfonic acid or p-toluenesulfonic acid.

The pharmaceutically acceptable salts can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th Edition, p. 1418, Mack Publishing Company, Easton, PA (1985).

Throughout the specification, groups and substituents thereof may be chosen by one skilled in the field to provide stable moieties and compounds and compounds useful as pharmaceutically-acceptable compounds and/or intermediate compounds useful in making pharmaceutically-acceptable compounds.

### EXAMPLES

The synthesis of the compounds of the present disclosure is illustrated in the following Schemes, using the compounds as disclosed in the working Examples as representatives.

A procedure for the synthesis of the unlabeled compounds of Formula (I) is outlined below. Scheme 1 describes the synthesis of fluoroalkyl carbamoyloxymethyltriazole cyclohexyl acids 8. The synthesis of the starting material, cyclohexyl ester triazole alcohols 1, has been described in US2017/0360759 (e.g. Examples 1E and 10F). Triazole alcohol 1 is reacted with 4-nitrophenyl chloroformate in the presence of an appropriate base to give the corresponding triazole 4-nitrophenyl carbonate 2. The triazole 4-nitrophenyl carbonate 2 is then reacted with an appropriate N-hydroxyalkyl amine 3 (n = 0-2) in the presence of an appropriate base to give the triazole N-hydroxylalkyl carbamate 4. The N-hydroxyalkyl carbamate 4 is reacted with an appropriate sulfonyl chloride 5 (e.g. p-toluenesulfonyl chloride) to give the corresponding sulfonate 6. Sulfonate 6 is reacted with an appropriate fluoride anion source (e.g. KF or Bu₄NF) to give the cyclohexyl ester triazole N-fluoroalkyl carbamate 7, which then undergoes ester deprotection to give the desired fluoroalkyl carbamoyloxymethyltriazole cyclohexyl acids 8.

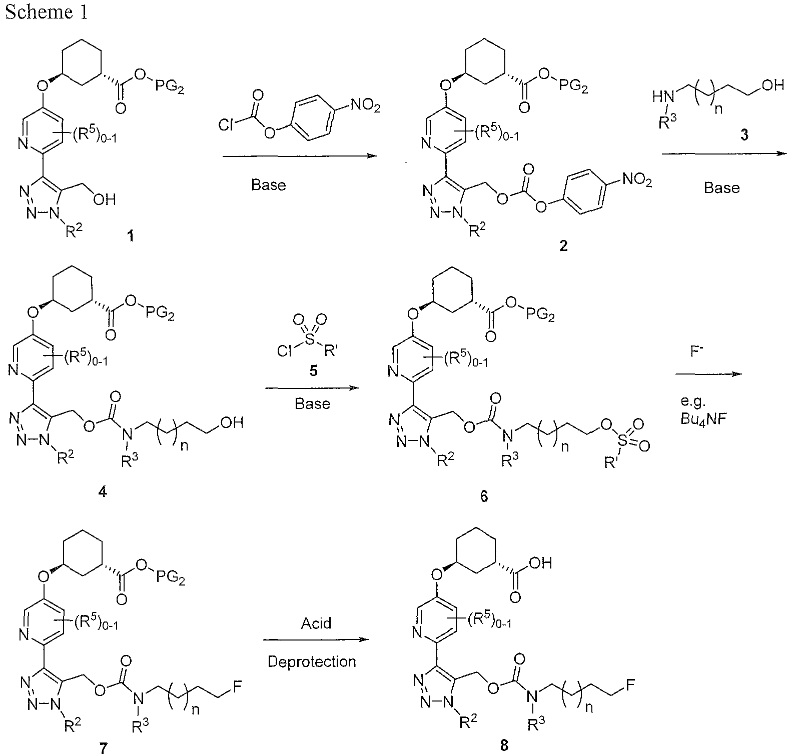

Scheme 2 describes the radiosynthesis of ¹⁸F-fluoroalkyl carbamoyloxymethyltriazole cyclohexyl acids. The radiosynthesis of the Sulfonate precursor 6 is reacted with an appropriate fluoride-18 anion source (e.g. K¹⁸F or Bu₄N¹⁸F), an appropriate phase transfer catalyst (e.g. Kryptofix^{®} 222) and an appropriate ionic liquid ( e.g. 1-butyl-3-methylimidazolium hexafluorophosphate) to give the ¹⁸F-cyclohexyl ester triazole N-fluoroalkyl carbamate 9, which then undergoes ester deprotection to give the desired ¹⁸F-fluoroalkyl carbamoyloxymethyltriazole cyclohexyl acids 10. 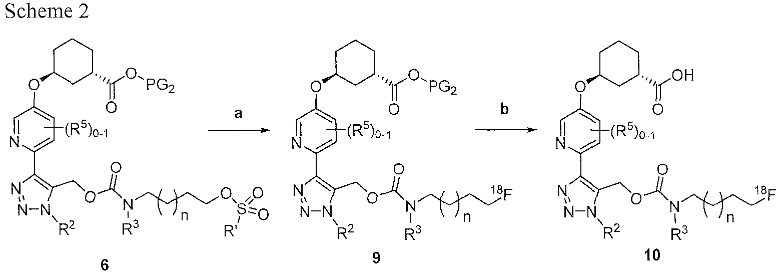 Reagents and Conditions:
a) K.2.2.2 K[¹⁸F], DMSO, 1-Buthyl-3-methylimidazolium hexafluorophosphate, 120 °C, 5 mins;
b) 2N NaOH, ethanol, 70 °C 10 mins

### HPLC Conditions:

Method A: Agilent 1100 series HPLC and Lab logic gamma ram radio-HPLC detector using the following method: Column: Zorbax SB C18, 4.6 x 250 mm, 3-µm particles; Mobile Phase: 60% acetonitrile in aqueous 0.1% trifluoroacetic acid; Flow: 1.00 mL/min; Detection: UV at 254 nm.

Method B: Column: Zorbax SB C18, 4.6 x 250 mm, 3-µm particles; Mobile Phase: 29% ethanol, 10% acetonitrile and 61% of 100 mM aqueous KH₂PO₄; Flow: 1.00 mL/min; Detection: UV at 254 nm.

Method C: Column: ChiralPak AD-H - 250 x 4.6 mm 5-µm particles; Mobile Phase: 15% isopropyl alcohol in heptane; Flow: 0.9 mL/min; Detection: UV at 254 nm.

### Example 1. Methyl (1S,3S)-3-((6-(5-(hydroxymethyl)-1-methyl-1H-1,2,3-triazol-4-yl)-2-methylpyridin-3-yl)oxy)cyclohexane-1-carboxylate

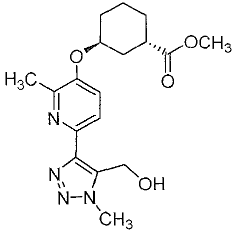

The title compound was prepared according to the synthetic sequences described for the preparation of either the corresponding isopropyl ester (Example 1E) or the ethyl ester (Example 10F) from the patent application US2017/0360759. The starting material used to prepare the title compound was (1S, 3R)-methyl 3-hydroxycyclohexane-1-carboxylate (rather than the corresponding cyclohexane isopropyl or ethyl esters).

### Example 2.Methyl (1R,3S)-3-((6-(5-(hydroxymethyl)-1-methyl-1H-1,2,3-triazol-4-yl)-2-methylpyridin-3-yl)oxy)cyclohexane-1-carboxylate

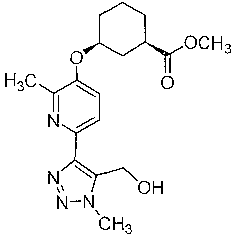

A clear solution of Example 1 compound (1.95g, 5.41 mmol) in MeOH (38.6 mL) was added a 5.4 M solution of NaOMe (4.0 mL, 21.64 mmol). The reaction was heated at 60 °C for 6 h and then cooled to RT. The reaction was placed in an ice bath, neutralized with 1 N HCl and then partially concentrated to remove the MeOH. The resulting cloudy mixture was partitioned between 1.0 M K₂HPO₄ and EtOAc and the layers were separated. The aqueous layer was extracted with EtOAc (1x). The organic layers were combined and washed with brine, dried (Na₂SO₄), filtered and concentrated to give a white solid weighing 1.66 g. Purification by chiral SFC Prep (Column: Chiralpak IA, 21 x 250 mm, 5 micron; Mobile Phase: 20% MeOH / 80% CO₂; Flow Conditions: 85 mL/min, 150 Bar, 40°C; Detector Wavelength: 254 nm) gave the second eluting diastereomer as the title compound (740 mg, 38% yield) as a white solid. Chiral analytical HPLC (Column: Chiralpak IA, 4.6 x 250 mm, 5 micron; Mobile Phase: 25%MeOH / 80% CO₂; Flow Conditions: 2.0 mL/min, 150 Bar, 40°C; Detector Wavelength: 254 nm) indicated 98.2% de [0.9:99.1 trans (4.33 min):cis (5.48 min)] at the methyl ester stereocenter. LCMS, [M + H]⁺ = 361.1. ¹H NMR (500 MHz, CDCl₃) δ 8.10 (d, J=8.8 Hz, 1H), 7.61 - 7.45 (m, 1H), 7.29 (d, J=8.8 Hz, 1H), 4.83 (s, 2H), 4.33 - 4.21 (m, 1H), 4.09 (s, 3H), 3.70 (s, 3H), 2.53 - 2.45 (m, 4H), 2.44 - 2.38 (m, 1H), 2.23 - 2.13 (m, 1H), 2.06 - 1.96 (m, 2H), 1.76 - 1.59 (m, 1H), 1.56 - 1.39 (m, 3H).

### Example 3. Methyl (1S,3S)-3-((2-methyl-6-(1-methyl-5-((((4-nitrophenoxy) carbonyl)oxy) methyl)-1H-1,2,3-triazol-4-yl)pyridin-3-yl)oxy)cyclohexane-1-carboxylate

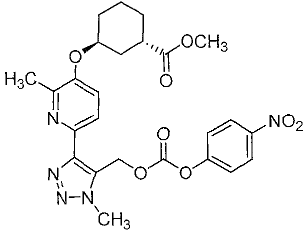

To a 0 °C solution of Example 1 compound (970 mg, 2.69 mmol) and pyridine (1.09 mL, 13.5 mmol) in DCM (17.9 mL) was added dropwise over 1 h a solution of 4-nitrophenyl chloroformate (1.09 g, 5.38 mmol) in DCM (3 mL). The reaction was then allowed to warm to RT and stirred at RT for 20 h, then was concentrated in vacuo to give a solid. A minimum amount of DCM was added to give a suspension and the solid (pyridine hydrochloride) was removed by filtration. The filtrate was concentrated in vacuo. The residue was chromatographed (120 g SiO₂ column; continuous gradient from 0-50% EtOAc in hexane) to give the title compound (1.12 g, 79 % yield) as a pale yellow solid. LCMS, [M + H]⁺ = 526.3. ¹H NMR (500 MHz, CDCl₃) δ 8.33 - 8.28 (m, 2H), 8.03 (d, *J*=8.5 Hz, 1H), 7.43 - 7.38 (m, 2H), 7.23 (d, *J*=8.5 Hz, 1H), 6.06 (s, 2H), 4.76 - 4.71 (m, 1H), 4.22 (s, 3H), 3.72 (s, 3H), 2.89 - 2.80 (m, 1H), 2.51 (s, 3H), 2.21 - 2.12 (m, 1H), 2.04 - 1.89 (m, 3H), 1.83 - 1.72 (m, 1H), 1.71 - 1.61 (m, 3H).

### Example 4. Methyl (1R,3S)-3-((2-methyl-6-(1-methyl-5-((((4-nitrophenoxy)carbonyl)oxy) methyl)-1H-1,2,3-triazol-4-yl)pyridin-3-yl)oxy)cyclohexane-1-carboxylate

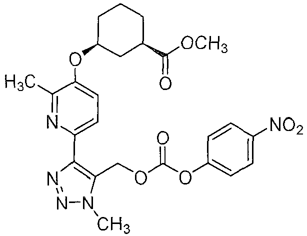

The title compound was prepared following the procedure as described for the synthesis of Example 3 compound by using Example 2 compound for the reaction with 4-nitrophenyl chloroformate (rather than Example 1 compound). LCMS, [M + H]⁺ = 526.1. ¹H NMR (500 MHz, CDCl₃) δ 8.33 - 8.27 (m, 2H), 8.02 (d, *J*=8.5 Hz, 1H), 7.43 - 7.38 (m, 2H), 7.22 (d, *J*=8.5 Hz, 1H), 6.06 (s, 2H), 4.32 - 4.19 (m, 4H), 3.70 (s, 3H), 2.54 - 2.45 (m, 4H), 2.45 - 2.36 (m, 1H), 2.22 - 2.12 (m, 1H), 2.05 - 1.95 (m, 2H), 1.77 - 1.64 (m, 1H), 1.55 - 1.38 (m, 3H).

### Example 5: Methyl (1S,3S)-3-((6-(5-((((4-hydroxybutyl)(methyl)carbamoyl)oxy) methyl)-1-methyl-1H-1,2,3-triazol-4-yl)-2-methylpyridin-3-yl)oxy)cyclohexane-1-carboxylate

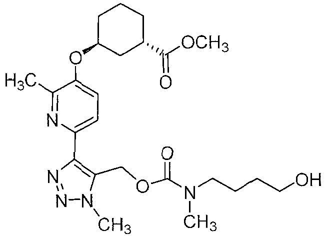

To a RT solution of Example 3 compound (1.90 g, 3.62 mmol) in THF (18.1 mL) was added 4-(methylamino)butan-1-ol (0.448 g, 4.34 mmol) followed by DIEA (0.76 mL, 4.34 mmol). After stirring for 18 h at RT, the reaction was concentrated in vacuo. The crude product was chromatographed (120 g SiO₂ column; continuous gradient from 0-10% MeOH in DCM over 20 min, then isocratic 10% MeOH in DCM for 20 min) to give the title compound (2.1 g, 119 % yield) as a yellow oil. The byproduct from the reaction, 4-nitrophenol, was also present. This material was used in the next step without further purification. LCMS, [M + H]⁺ = 490.3. ¹H NMR (500 MHz, CDCl₃) δ 7.96 (d, *J*=8.5 Hz, 1H), 7.21 (d, *J*=8.5 Hz, 1H), 5.84 - 5.71 (m, 2H), 4.74 - 4.69 (m, 1H), 4.15 (s, 3H), 3.77 - 3.65 (m, 4H), 3.54 - 3.45 (m, 1H), 3.41 - 3.31 (m, 1H), 3.27 - 3.16 (m, 1H), 2.99 - 2.80 (m, 4H), 2.52 (s, 3H), 2.22 - 2.11 (m, 1H), 2.05 - 1.87 (m, 3H), 1.84 - 1.73 (m, 1H), 1.71 - 1.36 (m, 8H). Some peaks appear to be rotameric.

### Example 6. Methyl (1S,3S)-3-((2-methyl-6-(1-methyl-5-(((methyl(4-(tosyloxy) butyl) carbamoyl)oxy)methyl)-1H-1,2,3-triazol-4-yl)pyridin-3-yl)oxy)cyclohexane-1-carboxylate

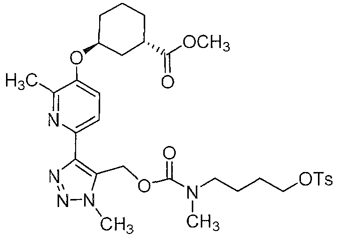

To a cooled (0 °C) solution of Example 5 compound (115 mg, 0.24 mmol), DMAP (2.9 mg, 0.023 mmol), and TEA (72.0 µL, 0.52 mmol) in DCM (2.35 mL) was added p-TsCl (53.7 mg, 0.28 mmol). The resulting reaction was allowed to warm to RT and stirred at RT overnight. The reaction was partitioned between water (3 mL) and Et₂O (3 mL) and the layers were separated. The aqueous layer was extracted with Et₂O (1x). The combined organic layers were dried (MgSO₄) and concentrated in vacuo. The crude product was chromatographed (24 g SiO₂ column; continuous gradient from 0-60% EtOAc in hexane over 25 min, then isocratic 60% EtOAc in hexane for 30 min) to give the title compound (126 mg, 83 % yield) as a clear, colorless residue. LCMS, [M + H]⁺ = 644.2. ¹H NMR (500 MHz, CDCl₃) δ 7.95 (d, *J*=8.5 Hz, 1H), 7.84 - 7.69 (m, 2H), 7.38 - 7.31 (m, 2H), 7.20 (d, *J*=8.5 Hz, 1H), 5.81 - 5.69 (m, 2H), 4.74 - 4.67 (m, 1H), 4.13 (s, 3H), 4.09 - 4.03 (m, 1H), 3.90 - 3.84 (m, 1H), 3.70 (s, 3H), 3.31 - 3.22 (m, 1H), 3.18 - 3.11 (m, 1H), 2.91 - 2.76 (m, 4H), 2.52 - 2.47 (m, 3H), 2.45 (s, 3H), 2.20 - 2.10 (m, 1H), 2.03 - 1.86 (m, 3H), 1.83 - 1.71 (m, 1H), 1.70 - 1.56 (m, 5H), 1.51 - 1.41 (m, 2H). Some peaks appear to be rotameric.

### Example 7. Methyl (1S,3S)-3-((2-methyl-6-(1-methyl-5-(((methyl(4-(((4-nitrophenyl)sulfonyl) oxy)butyl)carbamoyl)oxy)methyl)-1H-1,2,3-triazol-4-yl)pyridin-3-yl)oxy) cyclohexane-1-carboxylate

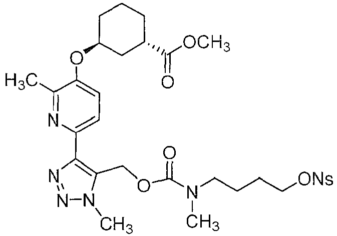

To a 0 °C solution of Example 5 compound (90 mg, 0.18 mmol), DMAP (2.2 mg, 0.018 mmol), and TEA (56 µL, 0.40 mmol) in DCM (1.8 mL) was added NsCl (45 mg, 0.20 mmol). The resulting reaction was allowed to slowly warm to RT. After 2 h stirring at RT, the reaction was partitioned between water (3 mL) and Et₂O (3 mL) and the layers were separated. The aqueous layer was extracted with Et₂O (1x). The combined organic layers were dried (MgSO₄), filtered and concentrated in vacuo. The crude product was chromatographed (12 g SiO₂ column; continuous gradient from 0-60% EtOAc in hexane) to give the title compound (25 mg, 19% yield) as a yellow solid. LCMS, [M + H]⁺ = 675.2. ¹H NMR (500 MHz, CDCl₃) δ 8.46 - 8.38 (m, 2H), 8.14 (br d, *J*=8.0 Hz, 1H), 8.08 - 8.00 (m, 1H), 7.97 (d, *J*=8.5 Hz, 1H), 7.22 (d, *J*=8.5 Hz, 1H), 5.81 - 5.70 (m, 2H), 4.76 - 4.69 (m, 1H), 4.24 - 4.17 (m, 1H), 4.15 (s, 3H), 3.98 - 3.91 (m, 1H), 3.72 (s, 3H), 3.34 - 3.26 (m, 1H), 3.24 - 3.13 (m, 1H), 2.96 - 2.78 (m, 4H), 2.53 - 2.46 (m, 3H), 2.22 - 2.11 (m, 1H), 2.05 - 1.88 (m, 3H), 1.85 - 1.46 (m, 8H). Some peaks appear to be rotameric.

### Example 8. Isopropyl (1S,3S)-3-((6-(5-(hydroxymethyl)-1-methyl-1H-1,2,3-triazol-4-yl)-2-methylpyridin-3-yl)oxy)cyclohexane-1-carboxylate

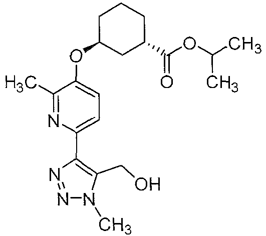

The title compound was prepared according to the experimental procedure described in US2017/0360759, Example 1E.

### Example 9. Isopropyl (1S,3S)-3-((2-methyl-6-(1-methyl-5-((((4-nitrophenoxy) carbonyl)oxy) methyl)-1H-1,2,3-triazol-4-yl)pyridin-3-yl)oxy)cyclohexane-1-carboxylate

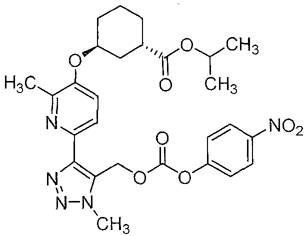

The title compound was prepared according to the experimental procedure described in US2017/0360759, Example 1F.

### Example 10. Preparation of Isopropyl (1S,3S)-3-((6-(5-((((4-hydroxybutyl) (methyl)carbamoyl) oxy)methyl)-1-methyl-1H-1,2,3-triazol-4-yl)-2-methylpyridin-3-yl)oxy)cyclohexane-1-carboxylate

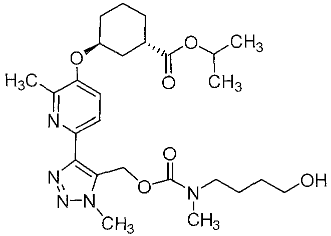

The title compound was prepared following the procedure as described for the synthesis of Example 5 compound by using Example 9 compound for the reaction with 4-(methylamino) butan-1-ol (rather than Example 3 compound). LCMS, [M + H]⁺ = 518.3. ¹H NMR (500 MHz, CDCl₃) δ 7.96 (d, *J*=8.5 Hz, 1H), 7.23 (d, *J*=8.5 Hz, 1H), 5.85 - 5.71 (m, 2H), 5.10 - 4.99 (m, 1H), 4.76 - 4.61 (m, 1H), 4.15 (br s, 3H), 3.78 - 3.63 (m, 1H), 3.55 - 3.44 (m, 1H), 3.41 - 3.30 (m, 1H), 3.26 - 3.14 (m, 1H), 2.99 - 2.82 (m, 3H), 2.82 - 2.73 (m, 1H), 2.52 (s, 3H), 2.14 - 2.07 (m, 1H), 2.02 - 1.86 (m, 3H), 1.86 - 1.35 (m, 9H), 1.32 - 1.20 (m, 6H). Some peaks appear to be rotameric.

### Example 11. Isopropyl (1S,3S)-3-((2-methyl-6-(1-methyl-5-(((methyl(4-(tosyloxy)butyl) carbamoyl)oxy)methyl)-1H-1,2,3-triazol-4-yl)pyridin-3-yl)oxy)cyclohexane-1-carboxylate

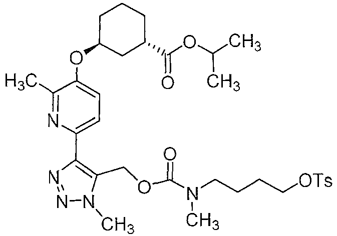

To a cooled (0 °C) solution of Example 10 compound (180 mg, 0.35 mmol) in pyridine (5 mL) was added p-TsCl (80 mg, 0.42 mmol). The resulting reaction was allowed to warm to RT, then was stirred at RT for 23 h. The reaction was partitioned between water (3 mL) and Et₂O (3 mL) and the layers were separated. The aqueous layer was extracted with Et₂O (1x). The combined organic layers were dried (MgSO₄), and concentrated in vacuo. The crude material was chromatographed (24 g SiO₂; continuous gradient from 0-100% EtOAc in hexane) to give the title compound (75 mg, 32 % yield) as a white gummy residue. LCMS, [M + H]⁺ = 672.3. ¹H NMR (500 MHz, CDCl₃) δ 7.94 (d, *J*=8.3 Hz, 1H), 7.83 - 7.67 (m, 2H), 7.38 - 7.30 (m, 2H), 7.21 (d, *J*=8.5 Hz, 1H), 5.80 - 5.68 (m, 2H), 5.08 - 4.96 (m, 1H), 4.72 - 4.64 (m, 1H), 4.12 (s, 3H), 4.08 - 4.01 (m, 1H), 3.91 - 3.82 (m, 1H), 3.31 - 3.21 (m, 1H), 3.18 - 3.08 (m, 1H), 2.91 - 2.72 (m, 4H), 2.52 - 2.46 (m, 3H), 2.44 (s, 3H), 2.12 - 2.04 (m, 1H), 2.00 - 1.86 (m, 3H), 1.81 - 1.70 (m, 1H), 1.70 - 1.55 (m, 5H), 1.50 - 1.40 (m, 2H), 1.27 - 1.22 (m, 6H). Some peaks appear to be rotameric.

### Example 12. Methyl (1S,3S)-3-((6-(5-((((4-fluorobutyl)(methyl)carbamoyl)oxy) methyl)-1-methyl-1H-1,2,3-triazol-4-yl)-2-methylpyridin-3-yl)oxy)cyclohexane-1-carboxylate

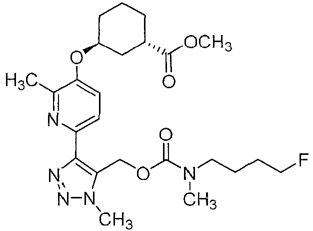

To a suspension of Example 3 compound (558 mg, 1.06 mmol) and 4-fluoro-N-methylbutan-1-amine-HCl salt (226 mg, 1.59 mmol) in THF (2.70 mL) and DCM (2.70 mL) was added dropwise TEA (0.59 mL, 4.25 mmol). The resulting yellow suspension was stirred at RT. Additional THF (2.70 mL) and DCM (2.70 mL) were added to facilitate mixing. After 1 h stirring at RT, the reaction mixture was diluted with EtOAc and washed with 1.0 M aq. K₂HPO₄ (3x), brine (1x), dried (Na₂SO₄), filtered and concentrated in vacuo to give a clear, yellow oil. This material was chromatographed (120 g SiO₂ column; continuous gradient from 0-100% EtOAc in Hexane) to give the title compound (440 mg, 84 % yield) as a clear, colorless viscous oil. LCMS, [M + H]⁺ = 492.2. ¹H NMR (500 MHz, CDCl₃) δ 7.97 (d, *J*=8.5 Hz, 1H), 7.21 (d, *J*=8.5 Hz, 1H), 5.78 (br d, *J*=12.7 Hz, 2H), 4.75 - 4.69 (m, 1H), 4.58 - 4.40 (m, 1H), 4.38 - 4.21 (m, 1H), 4.16 (br s, 3H), 3.71 (s, 3H), 3.42 - 3.31 (m, 1H), 3.27 - 3.16 (m, 1H), 2.97 - 2.81 (m, 4H), 2.52 (s, 3H), 2.22 - 2.13 (m, 1H), 2.05 - 1.88 (m, 3H), 1.84 - 1.46 (m, 8H). Some peaks appear rotameric. ¹⁹F NMR (471 MHz, CDCl₃) δ -218.58.

### Example 12 (Alternative Preparation). Methyl (1S,3S)-3-((6-(5-((((4-fluorobutyl)(methyl) carbamoyl)oxy)methyl)-1-methyl-1H-1,2,3-triazol-4-yl)-2-methylpyridin-3-yl)oxy)cyclohexane-1-carboxylate

A clear, pale yellow solution of Example 6 compound (79 mg, 0.12 mmol) in TBAF (1.0 M solution in THF, 2.45 mL, 2.45 mmol) was stirred at RT for 1.5 h, then was partitioned between water and Et₂O and the layers were separated. The organic layer was washed with brine, dried (Na₂SO₄), filtered and concentrated in vacuo to give a clear residue. The crude product was chromatographed (12 g SiO₂ column; continuous gradient from 0-60% EtOAc in hexane) to give the title compound (0.0354 g, 58 % yield) as a clear, colorless oil. LCMS, [M + H]⁺ = 492.4. ¹H NMR (500 MHz, CDCl₃) δ 7.97 (d, *J*=8.5 Hz, 1H), 7.21 (d, *J*=8.5 Hz, 1H), 5.78 (br d, *J*=12.7 Hz, 2H), 4.75 - 4.69 (m, 1H), 4.58 - 4.40 (m, 1H), 4.38 - 4.21 (m, 1H), 4.16 (br s, 3H), 3.71 (s, 3H), 3.42 - 3.31 (m, 1H), 3.27 - 3.16 (m, 1H), 2.97 - 2.81 (m, 4H), 2.52 (s, 3H), 2.22 - 2.13 (m, 1H), 2.05 - 1.88 (m, 3H), 1.84 - 1.46 (m, 8H). Some peaks appear to be rotameric. ¹⁹F NMR (471 MHz, CDCl₃) δ -218.58.

### Example 13. Methyl (1R,3S)-3-((6-(5-((((4-fluorobutyl)(methyl)carbamoyl)oxy) methyl)-1-methyl-1H-1,2,3-triazol-4-yl)-2-methylpyridin-3-yl)oxy)cyclohexane-1-carboxylate

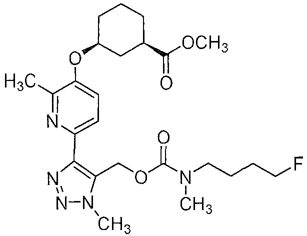

The title compound was prepared following the procedure as described for the synthesis of Example 12 compound by using Example 4 compound for the reaction with 4-fluoro-N-methylbutan-1-amine-HCl (rather than Example 3 compound). LCMS, [M + H]⁺ = 492.3. ¹H NMR (500 MHz, CDCl₃) δ 7.97 (d, *J*=8.3 Hz, 1H), 7.20 (d, *J*=8.5 Hz, 1H), 5.78 (br d, *J*=12.1 Hz, 2H), 4.58 - 4.40 (m, 1H), 4.37 - 4.20 (m, 2H), 4.16 (s, 3H), 3.70 (s, 3H), 3.41 - 3.30 (m, 1H), 3.27 - 3.16 (m, 1H), 2.98 - 2.81 (m, 3H), 2.53 - 2.44 (m, 4H), 2.44 - 2.37 (m, 1H), 2.22 - 2.12 (m, 1H), 2.05 - 1.95 (m, 2H), 1.78 - 1.64 (m, 3H), 1.58 - 1.40 (m, 5H).

### Example 14. (1S,3S)-3-((6-(5-((((4-Fluorobutyl)(methyl)carbamoyl)oxy)methyl)-1-methyl-1H-1,2,3-triazol-4-yl)-2-methylpyridin-3-yl)oxy)cyclohexane-1-carboxylic acid

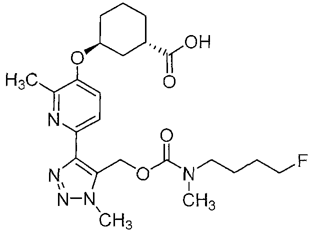

To a solution of Example 12 compound (0.300 g, 0.610 mmol) in THF (4.1 mL) was added 1.0 M aq. LiOH (3.0 mL, 3.0 mmol) at RT. The reaction was stirred at RT for 19 h, then was acidified with 1N aq. HCl to pH ~ 4 to 5 and then extracted with EtOAc (2x). The combined organic layers were washed with brine, dried (Na₂SO₄), filtered and concentrated in vacuo to give a clear, colorless residue. The crude product was chromatographed (80 g SiO₂ column, continuous gradient from 0-10% MeOH in DCM) to give, following lyophilization, the title compound (241 mg, 82 % yield) as a white solid. LCMS, [M + H]⁺ = 478.2. ¹H NMR (500 MHz, DMSO-d₆) δ 12.21 (br s, 1H), 7.85 (d, *J*=8.5 Hz, 1H), 7.48 (d, *J*=8.8 Hz, 1H), 5.65 (br d, *J*=13.5 Hz, 2H), 4.82 - 4.76 (m, 1H), 4.53 - 4.36 (m, 1H), 4.31 - 4.15 (m, 1H), 4.10 (s, 3H), 3.27 - 3.20 (m, 1H), 3.16 - 3.09 (m, 1H), 2.85 - 2.73 (m, 3H), 2.68 - 2.59 (m, 1H), 2.42 (s, 3H), 2.08 - 1.96 (m, 1H), 1.92 - 1.74 (m, 3H), 1.69 - 1.34 (m, 8H). Some peaks appear to be rotameric. ¹⁹F NMR (471 MHz, DMSO-d₆) δ -216.79, -216.84. Chiral analytical HPLC (Chiralpak OJ-H, 4.6 x 250 mm, 5 micron. Mobile Phase: 10% MeOH / 90% CO₂. Flow Conditions: 2 mL/min, 150 Bar, 40°C. Detector Wavelength: 220 nm) indicated 97% de [98.6:1.4 trans (8.61 min):cis (11.58 min)] at the carboxylic acid stereocenter. (see. US2017/0360759 as Example 192)

### Example 14 (Alternative Preparation). (1S,3S)-3-((6-(5-((((4-Fluorobutyl)(methyl) carbamoyl) oxy)methyl)-1-methyl-1H-1,2,3-triazol-4-yl)-2-methylpyridin-3-yl)oxy)cyclohexane-1-carboxylic acid, trifluoroacetic acid salt

To a solution of Example 12 (Alternative Preparation; 0.023 g, 0.046 mmol) in THF (0.31 mL) was added 1.0 M aq. LiOH (0.23 mL, 0.23 mmol) at RT. The reaction was stirred at RT for 22 h, then was concentrated in vacuo to remove the THF. The residue was dissolved in water and MeCN and acidified with TFA. This material was purified by preparative HPLC (Column: Sunfire Prep C18 OBD 5 µm; 30 x 100 mm. Solvent A: 10:90:0.1 MeCN:H₂O:TFA; Solvent B: 90:10:0.1 MeCN:H₂O:TFA. Flow rate of 40 mL/minute using gradient elution 10-100% solvent B while the UV was monitored at 220 nm) to give, following lyophilization, the title compound as the TFA salt (0.0195 g, 71 % yield) as a white solid. LCMS, [M + H]⁺ = 478.4. ¹H NMR (500 MHz, DMSO-d₆ and D₂O) δ 7.85 (d, *J*=8.5 Hz, 1H), 7.50 (br d, *J*=8.5 Hz, 1H), 5.64 (br d, *J*=12.4 Hz, 2H), 4.81 - 4.77 (m, 1H), 4.53 - 4.36 (m, 1H), 4.31 - 4.15 (m, 1H), 4.10 (s, 3H), 3.28 - 3.18 (m, 1H), 3.16 - 3.05 (m, 1H), 2.84 - 2.72 (m, 3H), 2.67 - 2.61 (m, 1H), 2.43 (s, 3H), 2.08 - 1.99 (m, 1H), 1.90 - 1.75 (m, 3H), 1.70 - 1.35 (m, 8H). Some peaks appear to be rotameric. ¹⁹F NMR (471 MHz, DMSO-d₆ and D₂O) δ -74.66, -216.78, - 216.84. Chiral analytical HPLC (Chiralpak ID, 4.6 x 250 mm, 5 micron. Mobile Phase: 20% MeOH / 80% CO₂. Flow Conditions: 2 mL/min, 150 Bar, 40°C. Detector Wavelength: 220 nm) indicated 83% de [91.6:8.4 trans (8.47 min):cis (9.56 min)] at the carboxylic acid stereocenter.

### Example 15. (1R,3S)-3-((6-(5-((((4-Fluorobutyl)(methyl)carbamoyl)oxy)methyl)-1-methyl-1H-1,2,3-triazol-4-yl)-2-methylpyridin-3-yl)oxy)cyclohexane-1-carboxylic acid

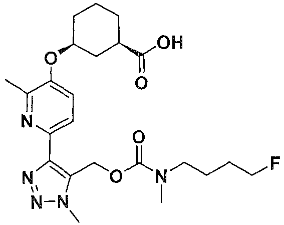

The title compound was prepared following the procedure as described for the synthesis of Example 14 compound by using Example 13 compound for the reaction with LiOH (rather than Example 12 compound). LCMS, [M + H]⁺ = 478.2. ¹H NMR (500 MHz, DMSO-d₆) δ 12.28 - 12.14 (m, 1H), 7.84 (d, *J*=8.5 Hz, 1H), 7.53 (d, *J*=8.5 Hz, 1H), 5.69 - 5.60 (m, 2H), 4.54 - 4.35 (m, 2H), 4.32 - 4.14 (m, 1H), 4.10 (s, 3H), 3.28 - 3.09 (m, 2H), 2.84 - 2.71 (m, 3H), 2.48 - 2.39 (m, 1H), 2.37 (s, 3H), 2.36 - 2.19 (m, 1H), 2.11 - 2.02 (m, 1H), 1.92 - 1.79 (m, 2H), 1.67 - 1.50 (m, 2H), 1.50 - 1.23 (m, 6H). ¹⁹F NMR (377 MHz, DMSO-d₆) δ -216.80, -216.84. Some peaks appear to be rotameric. Chiral analytical HPLC (Chiralpak OJ-H, 4.6 x 250 mm, 5 micron. Mobile Phase: 10% MeOH / 90% CO₂. Flow Conditions: 2 mL/min, 150 Bar, 40°C. Detector Wavelength: 220 nm) indicated >98% de [0.7:99.3 trans (8.61 min):cis (11.58 min)] at the carboxylic acid stereocenter.

### Example 16. Preparation of ¹⁸F-(1S,3S)-3-((6-(5-((((4-(fluoro)butyl)(methyl) carbamoyl)oxy)methyl)-1-methyl-1H-1,2,3-triazol-4-yl)-2-methylpyridin-3-yl)oxy)cyclohexane-1-carboxylic acid (Method A)

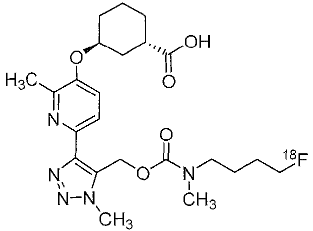

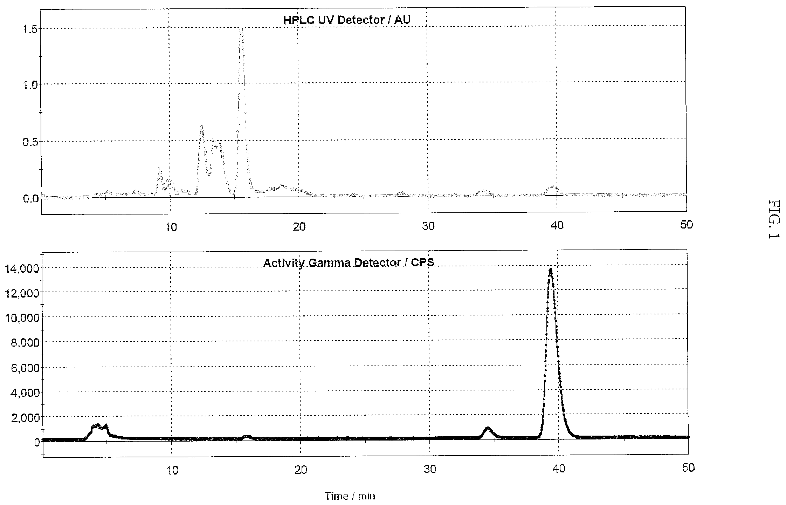

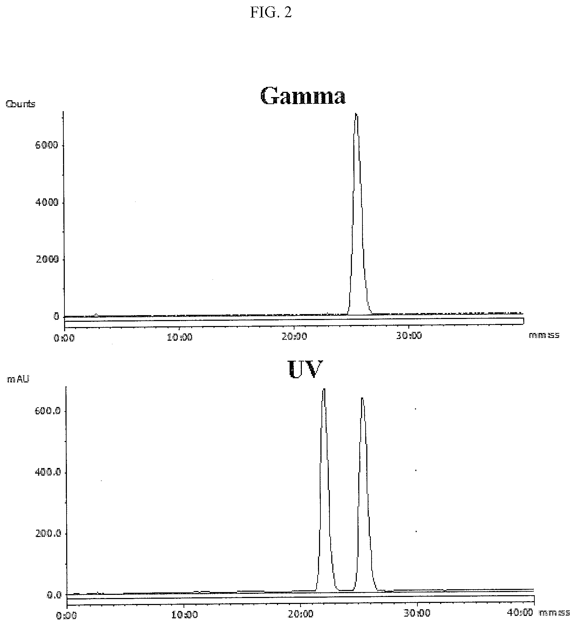

An aqueous ¹⁸F-Fluoride solution ( 2.0 ml, 111 GBq (3000 mCi)) was delivered to a QMA light solid phase extraction cartridge (the cartridge was pre-conditioned sequentially with 5ml of 0.5 M potassium bicarbonate, 5 ml of deionized water, and 5 ml of acetonitrile before use). Upon completion of this transfer, the aqueous [¹⁸F]-fluoride was released from the QMA by the addition of a mixture of potassium carbonate ( 2.0 mg in distilled water (DI), 0.1 ml), 4,7,13,16,21,24-hexaoxa-1,10-diazabicyclo[8.8.8]hexacosane (10 mg, 0.027 mmol) and 1.5 ml of acetonitrile. The solvent was evaporated under a gentle stream of nitrogen at 90 °C and vacuum to generate the K.2.2.2/K[¹⁸F]F complex. Upon completion of this process, 2 mg of Example 6 compound (methyl (1S,3S)-3-((2-methyl-6-(1-methyl-5-(((methyl(4-(tosyloxy)butyl)carbamoyl)oxy)methyl)-1H-1,2,3-triazol-4-yl)pyridin-3-yl)oxy)cyclohexane-1-carboxylate) (2 mg, 3.11 µmol) was dissolved in 0.7 ml DMSO and 1-butyl-3-methylimidazolium hexafluorophosphate (300 µl, 1.441 mmol) was added to the dried kyptand. This resultant solution was heated at 120 °C for 5 minutes. Upon completion of this heating period, the crude reaction mixture was cooled to 45 °C. Next, 4.0 ml of a solution that contained 37.5% acetonitrile dissolved in aqueous 0.1% trifluoroacetic acid was transferred into a dilution flask that contained 70 ml of DI water. After this transfer, the contents of the dilution flask were loaded onto a C18 Plus (360 mg) solid phase extraction cartridge. After this sample was completely transferred, the penultimate was released from the cartridge with 2 ml of ethanol into a synthesis reactor that contained 0.5 ml of 2 N NaOH. This resulting reaction was heated at 70 °C for 10 minutes. After this time, the reaction was cooled to 45 °C and to this reaction mixture was added 1 ml 1N HCl and 1.5 ml 50 mM potassium phosphate monobasic buffer (pH 5.0). This crude reaction mixture was loaded onto a Zorbax C18 9.6 x 250 mm HPLC column and purified using the following HPLC purification method: 29% Ethanol 10% acetonitrile in 100 mM potassium phosphate monobasic buffer @ pH 5.0 at 3.5 ml/min. 254 nm & pressure: 1700 PSI .The ¹⁸F-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid was isolated between the 38-41 minute mark of the chromatogram and this sample was collected into a dilution flask that contained 50 ml 250 mM Tris Buffer at pH 8.0 as shown in Figure 1. This solution was transferred to a C18 light (130 mg) solid phase extraction cartridge. This cartridge was pre-activated with 5 ml of ethanol followed by 10 ml of sterile water before the synthesis. After transfer, the cartridge was washed with 7 ml of 0.5 mg/ml sodium ascorbate pH 7.0, followed by 7 ml sterile water and then finally eluted with 2.0 ml of ethanol into the sterile product vial. 7.5 GBq (204 mCi) of ¹⁸F-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid was isolated and analyzed via reverse phase HPLC for chemical identity with co-injection of non-radioactive standard, radiochemical and chemical purity and specific activity as shown in Figure 2. The isolated product co-eluted with non-radioactive reference standard. The sample was 100 % radiochemically pure, 97% chemically pure, 100% diasteromeric excess and with a specific activity of 0.15 GBq (4 mCi)/nmol. Analytical reverse phase HPLC was used to determine structural identity, radiochemical purity and chemical purity using the following method: Zorbax SB-C18 - 250 x 4.6 mm - Sum semi-prep HPLC column using an isocratic method consisting of a solution of 29% ethanol, 10% acetonitrile and 61% of 100 mM aqueous KH₂PO₄ using a flow rate 1.0 ml/min while the UV was monitored at 254 nm. Retention time of the ¹⁸F-(1S,3S)-3-((6-(5-((((4-(fluoro)butyl)(methyl) carbamoyl)oxy)methyl)-1-methyl-1H-1,2,3-triazol-4-yl)-2-methylpyridin-3-yl)oxy)cyclohexane-1-carboxylic acid was 26.5 minutes. Diastereomeric excess was measured using an analytical chiral HPLC using the following parameters. ChiralPak AD-H - 250 x 4.6 mm column using an isocratic HPLC method using a solution of 15% isopropyl alcohol in heptane; at a flow rate 0.9 ml/min while the UV was monitored at 254 nm. Retention time of the ¹⁸F-(1S,3S)-3-((6-(5-((((4-(fluoro)butyl)(methyl) carbamoyl)oxy)methyl)-1-methyl-1H-1,2,3-triazol-4-yl)-2-methylpyridin-3-yl)oxy)cyclohexane-1-carboxylic acid was at 30 minutes. Specific activity was determined using a 4-point standard curve (analytical HPLC peak area (Y) versus standard concentration (X: in nmol). The fitted line equation was determined using reverse phase HPLC using the following parameters: Zorbax C18 - 250 x 4.6 - 5um HPLC column using a mobile phase of 60% acetonitrile in aqueous 0.1% TFA at a flow rate of 1.0 ml/min while monitoring the UV at 254 nm.

FIG. 1 shows the semi-preparative HPLC purification of the title compound.

FIG. 2 shows Co-injection of (1S,3S)-3-(6-(5-(((4-[18F]-fluorobutyl)(methyl) carbamoyloxy)methyl)-1-methyl-1H-1,2,3-triazol-4-yl)-2-methylpyridin-3-yloxy)cyclohexanecarboxylic acid and the mixture of reference standard of (1S,3S)-3-(6-(5-(((4-fluorobutyl)(methyl)carbamoyloxy)methyl)-1-methyl-1H-1,2,3-triazol-4-yl)-2-methylpyridin-3-yloxy)cyclohexanecarboxylic acid and (1R,3S)-3-(6-(5-(((4-fluorobutyl)(methyl)carbamoyloxy)methyl)-1-methyl-1H-1,2,3-triazol-4-yl)-2-methylpyridin-3-yloxy)cyclohexanecarboxylic acid using analytical reverse phase HPLC.

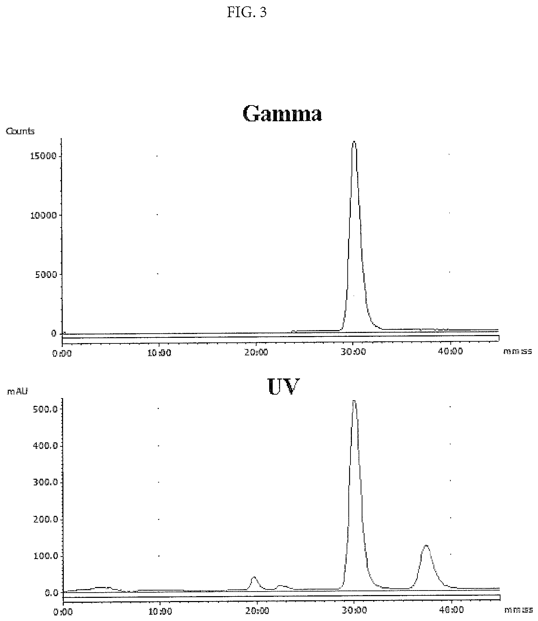

FIG. 3 shows Co-elution of (1S,3S)-3-(6-(5-(((4-[18F]-fluorobutyl)(methyl) carbamoyloxy)methyl)-1-methyl-1H-1,2,3-triazol-4-yl)-2-methylpyridin-3-yloxy)cyclohexanecarboxylic acid and the mixture of reference standard of (1S,3 S)-3-(6-(5-(((4-fluorobutyl)(methyl)carbamoyloxy)methyl)-1-methyl-1H-1,2,3-triazol-4-yl)-2-methylpyridin-3-yloxy)cyclohexanecarboxylic acid and (1R,3S)-3-(6-(5-(((4-fluorobutyl)(methyl)carbamoyloxy)methyl)-1-methyl-1H-1,2,3-triazol-4-yl)-2-methylpyridin-3-yloxy)cyclohexanecarboxylic acid using analytical chiral HPLC.

### Example 16 (Alternative Preparation). Synthesis of ¹⁸F-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid (Method B)

An aqueous ¹⁸F-Fluoride solution ( 2.0 ml, 111 GBq, 3000 mCi) was delivered to a QMA light solid phase extraction cartridge (the cartridge was pre-conditioned sequentially with 5ml of 0.5 M potassium bicarbonate, 5 ml of deionized water, and 5 ml of acetonitrile before use). Upon completion of this transfer, the aqueous ¹⁸F-fluoride was released from the QMA by the addition of a mixture of potassium carbonate ( 2.0 mg in distilled water (DI), 0.1 ml), 4,7,13,16,21,24-hexaoxa-1,10-diazabicyclo[8.8.8] hexacosane (10 mg, 0.027 mmol) and 1.5 ml of acetonitrile. The solvent was evaporated under a gentle stream of nitrogen at 90 °C and vacuum to generate the K.2.2.2/K[¹⁸F]F complex. Upon completion of this process, 2 mg of methyl (1S,3S)-3-((2-methyl-6-(1-methyl-5-(((methyl(4-(tosyloxy)butyl)carbamoyl)oxy)methyl)-1H-1,2,3-triazol-4-yl)pyridin-3-yl)oxy)cyclohexane-1-carboxylate (2 mg, 3.11 µmol) dissolved in 0.7 ml DMSO and 1-butyl-3-methylimidazolium hexafluorophosphate (300 µl, 1.441 mmol) was added to the dried kyptand. This resultant solution was heated at 120 °C for 5 minutes. Upon completion of this heating period, the crude reaction mixture was cooled to 45 °C. Next, 4.0 ml of an aqueous 0.1% trifluoroacetic acid solution was added to the crude reaction and this solution purified using a Zorbax C18 9.6 x 250 mm HPLC column using the following HPLC method: 50% acetonitrile in aqueous 0.1% trifluoroacetic acid solution at 5 ml/min. The penultimate was isolated between 7.5-8.5 minutes of the chromatogram and this sample was collected into a dilution flask that contained an additional 70 ml of aqueous 0.1% trifluoroacetic acid in DI water. This solution was then transferred to a C18 plus 360 mg solid phase extraction cartridge. After this transfer, the contents of the dilution flask were loaded onto a C18 Plus (360 mg) solid phase extraction cartridge. After loaded the penultimate was washed with 5 ml of DI water, followed by elution with 1 ml of acetonitrile into a solution of 1 ml of 2N NaOH and the resulting solution was heated at 70 °C 25 minutes. Upon the completion of this time, the crude reaction mixture was transferred to dilution flask that contained 10 ml of 2N NaOH and 40 ml of DI water. The contents of the dilution flask were then added to a C18 light (130 mg) solid phase extraction cartridge. Upon completion of the transfer, the cartridge was further washed with 10 ml of a 0.5 mg/ml solution of ascorbic acid, followed by 10 ml of sterile water for injection and finally the purified ¹⁸F-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid was released from the cartridge using 1 ml of ethnaol into a sterile vial. 7.8 GBq (212 mCi) of final product was isolated, the resulting solution was filtered through a 0.2 micron filter and diluted with 4 ml of saline. Chemical identity with co-injection of non-radioactive standard, radiochemical purity and chemical purity, specific activity was measured using analytical HPLC methods as previously described. The isolated product co-eluted with non-radioactive reference standard. The sample was 100 % radiochemically pure, 97% chemically pure and with a specific activity of 0.14 GBq (3.8 mCi) /nmol.

The ¹⁸F-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid can be used in a variety of *in vitro* and/or *in vivo* imaging applications, including diagnostic imaging, basic research, and radiotherapeutic applications. Specific examples of possible diagnostic imaging and radiotherapeutic applications, include determining the location, the relative activity and/or quantifying LPA1 positive tissues, radioimmunoassay of LPA1 positive tissues, and autoradiography to determine the distribution of LPA1 positive tissues in a mammal or an organ or tissue sample thereof. In particular, the ¹⁸F-(1S,3S)-3-((6-(5-((((4-(fluoro)butyl)(methyl)carbamoyl)oxy)methyl)-1-methyl-lH-1,2,3-triazol-4-yl)-2-methylpyridin-3-yl)oxy)cyclohexane-1-carboxylic acid is useful for positron emission tomographic (PET) imaging of LPA1 positive tumors in the lung, heart, kidneys, liver and skin and other organs of humans and experimental animals. PET imaging using the [¹⁸F]-(1S,3S)-3-((6-(5-((((4-(fluoro)butyl)(methyl) carbamoyl)oxy)methyl)-1-methyl-1H-1,2,3-triazol-4-yl)-2-methylpyridin-3-yl)oxy)cyclohexane-1-carboxylic acid can be used to obtain the following information: relationship between level of tissue occupancy by LPA1 antagonist candidate in medicaments and clinical efficacy in patients; dose selection for clinical trials of LPA1 treating medicaments prior to initiation of long term clinical studies; comparative potencies of structurally novel LPA1 treating medicaments; investigating the influence of LPA1 treating medicaments on *in vivo* transporter affinity and density during the treatment of clinical targets with LPA1 treating medicaments; changes in the density and distribution of LPA1 positive tissues during effective and ineffective treatment.

### Example 17. Preparation of [¹⁸F]-(1R,3S)-3-((6-(5-((((4-(fluoro)butyl)(methyl) carbamoyl)oxy)methyl)-1-methyl-1H-1,2,3-triazol-4-yl)-2-methylpyridin-3-yl)oxy)cyclohexane-1-carboxylic acid (Method A)

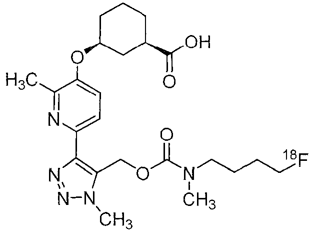

An aqueous ¹⁸F-Fluoride solution ( 2.0 ml, 111 GBq (3000 mCi)) was delivered to a QMA light solid phase extraction cartridge (the cartridge was pre-conditioned sequentially with 5ml of 0.5 M potassium bicarbonate, 5 ml of deionized water, and 5 ml of acetonitrile before use). Upon completion of this transfer, the aqueous [¹⁸F]-fluoride was released from the QMA by the addition of a mixture of potassium carbonate ( 2.0 mg in distilled water (DI), 0.1 ml), 4,7,13,16,21,24-hexaoxa-1,10-diazabicyclo[8.8.8]hexacosane (12 mg, 0.032 mmol) and 1.5 ml of acetonitrile. The solvent was evaporated under a gentle stream of nitrogen at 90 °C and vacuum to generate the K.2.2.2/K[¹⁸F]F complex. Upon completion of this process, 2 mg of methyl (1S,3S)-3-((2-methyl-6-(1-methyl-5-(((methyl(4-(tosyloxy)butyl)carbamoyl)oxy)methyl)-1H-1,2,3-triazol-4-yl)pyridin-3-yl)oxy)cyclohexane-1-carboxylate (2 mg, 3.11 µmol) was dissolved in 0.7 ml DMSO and 1-butyl-3-methylimidazolium hexafluorophosphate (300 µl, 1.441 mmol) was added to the dried kyptand. This resultant solution was heated at 120 °C for 15 minutes. Upon completion of this heating period, the crude reaction mixture was cooled to 45 °C. Next, 4.0 ml of a solution that contained 37.5% acetonitrile dissolved in aqueous 0.1% trifluoroacetic acid was transferred into a dilution flask that contained 70 ml of DI water. After this transfer, the contents of the dilution flask were loaded onto a C18 Plus (360 mg) solid phase extraction cartridge. After this sample was completely transferred, the penultimate was released from the cartridge with 2 ml of ethanol into a synthesis reactor that contained 0.5 ml of 2 N NaOH. This resulting reaction was heated at 70 °C for 10 minutes. After this time, the reaction was cooled to 45 °C and to this reaction mixture was added 1 ml 1N HCl and 1.5 ml 50 mM potassium phosphate monobasic buffer (pH 5.0). This crude reaction mixture was loaded onto a Zorbax C18 9.6 x 250 mm HPLC column and purified using the following HPLC purification method: 29% Ethanol 10% acetonitrile in 100 mM potassium phosphate monobasic buffer @ pH 5.0 at 3.5 ml/min. 254 nm & pressure: 1700 PSI .The [¹⁸F]-(1R,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid was isolated between the 28-31 minute mark of the chromatogram and this sample was collected into a dilution flask that contained 50 ml 250 mM Tris Buffer at pH 8.0. This solution was transferred to a C18 light (130 mg) solid phase extraction cartridge. This cartridge was pre-activated with 5 ml of ethanol followed by 10 ml of sterile water before the synthesis. After transfer, the cartridge was washed with 7 ml of 0.5 mg/ml sodium ascorbate pH 7.0, followed by 7 ml sterile water and then finally eluted with 2.0 ml of ethanol into the sterile product vial. 4.1 GBq (112 mCi) of [¹⁸F]-(1R,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid was isolated and analyzed via reverse phase HPLC for chemical identity with co-injection of non-radioactive standard, radiochemical and chemical purity and specific activity. The isolated product co-eluted with non-radioactive reference standard. The sample was 100 % radiochemically pure, 97% chemically pure, 100% diasteromeric excess and with a specific activity of 0.15 GBq (4 mCi)/nmol. Analytical reverse phase HPLC was used to determine structural identity, radiochemical purity and chemical purity using the following method: Zorbax SB-C18 - 250 x 4.6 mm - Sum semi-prep HPLC column using an isocratic method consisting of a solution of 29% ethanol, 10% acetonitrile and 61% of 100 mM aqueous KH₂PO₄ using a flow rate 1.0 ml/min while the UV was monitored at 254 nm. Retention time of the [¹⁸F]-(1R,3S)-3-((6-(5-((((4-(fluoro)butyl)(methyl) carbamoyl)oxy)methyl)-1-methyl-1H-1,2,3-triazol-4-yl)-2-methylpyridin-3-yl)oxy)cyclohexane-1-carboxylic acid was 26.5 minutes. Diastereomeric excess was measured using an analytical chiral HPLC using the following parameters. ChiralPak AD-H - 250 x 4.6 mm column using an isocratic HPLC method using a solution of 15% isopropyl alcohol in heptane; at a flow rate 0.9 ml/min while the UV was monitored at 254 nm. Retention time of the [¹⁸F]-(1R,3S)-3-((6-(5-((((4-(fluoro)butyl)(methyl) carbamoyl)oxy)methyl)-1-methyl-1H-1,2,3-triazol-4-yl)-2-methylpyridin-3-yl)oxy)cyclohexane-1-carboxylic acid was at 37.5 minutes. Specific activity was determined using a 4-point standard curve (analytical HPLC peak area (Y) versus standard concentration (X: in nmol). The fitted line equation was determined using reverse phase HPLC using the following parameters: Zorbax C18 - 250 x 4.6 - Sum HPLC column using a mobile phase of 60% acetonitrile in aqueous 0.1% TFA at a flow rate of 1.0 ml/min while monitoring the UV at 254 nm.

Alternatively, an aqueous ¹⁸F-Fluoride solution ( 2.0 ml, 111 GBq (3000 mCi)) was delivered to a QMA light solid phase extraction cartridge (the cartridge was pre-conditioned sequentially with 5ml of 0.5 M potassium bicarbonate, 5 ml of deionized water, and 5 ml of acetonitrile before use). Upon completion of this transfer, the aqueous [¹⁸F]-fluoride was released from the QMA by the addition of a mixture of potassium carbonate ( 1.0 mg in distilled water (DI), 0.1 ml), 4,7,13,16,21,24-hexaoxa-1,10-diazabicyclo[8.8.8]hexacosane (10 mg, 0.032 mmol) and 0.9 ml of acetonitrile. The solvent was evaporated under a gentle stream of nitrogen at 120 °C and vacuum to generate the K.2.2.2/K[¹⁸F]F complex. Upon completion of this process, 2 mg of methyl (1S,3S)-3-((2-methyl-6-(1-methyl-5-(((methyl(4-(tosyloxy)butyl)carbamoyl)oxy)methyl)-1H-1,2,3-triazol-4-yl)pyridin-3-yl)oxy)cyclohexane-1-carboxylate (2 mg, 3.11 µmol) was dissolved in 0.7 ml DMSO was added to the dried kyptand. This resultant solution was heated at 120 °C for 5 minutes. Upon completion of this heating period, the crude reaction mixture was cooled to 45 °C. Next, 4.0 ml of a solution that contained 37.5% acetonitrile dissolved in aqueous 0.1% trifluoroacetic acid was transferred into a dilution flask that contained 70 ml of DI water. After this transfer, the contents of the dilution flask were loaded onto a C18 Plus (360 mg) solid phase extraction cartridge. After this sample was completely transferred, the penultimate was released from the cartridge with 1.9 ml of ethanol into a synthesis reactor that contained 0.5 ml of 2 N NaOH. This resulting reaction was heated at 70 °C for 10 minutes. After this time, the reaction was cooled to 45 °C and to this reaction mixture was added 1 ml 1N HCl and 1.5 ml 50 mM potassium phosphate monobasic buffer ( pH 5.0). This crude reaction mixture was loaded onto a Zorbax C18 9.6 x 250 mm HPLC column and purified using the following HPLC purification method: 29% Ethanol 10% acetonitrile in 100 mM potassium phosphate monobasic buffer @ pH 5.0 at 3.5 ml/min. 254 nm & pressure: 1700 PSI .The [¹⁸F]-(1R,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid was isolated between the 28-31 minute mark of the chromatogram and this sample was collected into a dilution flask that contained 50 ml 250 mM Tris Buffer at pH 8.0 200 mg sodium ascorbate. This solution was transferred to a C18 light (130 mg) solid phase extraction cartridge. This cartridge was pre-activated with 5 ml of ethanol followed by 10 ml of sterile water before the synthesis. After transfer, the cartridge was washed with 7 ml of 1 mg/ml sodium ascorbate pH 7.0, eluted with 1.0 ml of ethanol followed by 7 ml of saline conatining 7 mg of sodium ascrobate through a 0.22 micron filter into the sterile product vial. 3.6 GBq (97 mCi) of [¹⁸F]-(1R,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid was isolated and analyzed via reverse phase HPLC for chemical identity with co-injection of non-radioactive standard, radiochemical and chemical purity and specific activity. The isolated product co-eluted with non-radioactive reference standard. The sample was 100 % radiochemically pure, 97% chemically pure, 100% diasteromeric excess and with a specific activity of 0.15 GBq (4 mCi)/nmol. Analytical reverse phase HPLC was used to determine structural identity, radiochemical purity and chemical purity using the following method: column: Gemini NX C18, 5 µm, 4.6 x 250 mm; mobile phase: 37% acetonitrile and 63% 0.1 M ammonium formate containing 0.5% acetic acid, pH 4.2; flow rate: 2 mL/min; while the UV was monitored at 251 nm.

### Example 18. In-vitro whole cell binding of [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid

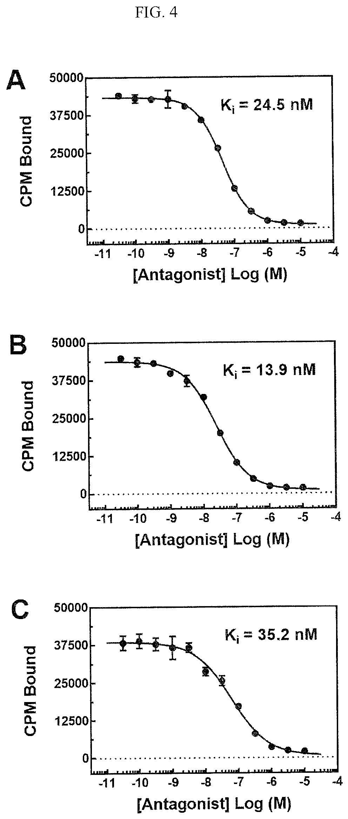

The effectiveness of compounds of the present invention as LPA1 inhibitors can be determined in an LPA1 binding assay as follows: Chinese hamster ovary cells overexpressing human LPA1 were maintained in F12 medium (Gibco #11765) supplemented with 10% fetal bovine serum and 1 mg/ml hygromycin (Invitrogen #10687-010). On day of assay, 150 cm² flasks of subconfluent cells were harvested with TripLE Express Dissociation reagent (Gibco #12605-010), counted, centrifuged, and resuspended in ice-cold Binding Assay Buffer (BAB: 50mM HEPES pH 7.2, 100mM NaCl, 2mM EDTA) at 2 x 10⁶ cells per ml. Cells were kept on ice until use. Test compound dilution plates were prepared by serially diluting test compounds (2mM stock in 100% DMSO, 1:3.26 dilutions) in DMSO, then diluting 1:50 in BAB to generate twelve half-log test concentrations at 4X final concentration. Binding assay were carried out in non -binding surface 96-well plates (Corning #3605) at room temperature for 1 hour. Assays were constructed as follows: 50µl of 4X compound and 75µl of ¹⁸F tracer (4000 Ci/mmol, final assay concentration 3.8nM) were added to each well, followed by assay initiation by addition of 75µl cell suspension (150000 cells total per well). After incubation at room temperature for 1 hr, plates were harvested by vacuum filtration on Unifilter-96 GF/B filter plates (Perkin-Elmer #6005177, pre-wet with 0.3% polyethylenimine in water). Filter platers were washed 3 x with 350µl of PBS/0.01% Triton X-100 and air dried for 20min. Individual filter discs were cut out of the filter plate and counted in a gamma counter. IC₅₀ values were determined by fitting the data to a 4-parameter logistic equation (GraphPad Prism, San Diego CA). FIG. 4 shows that the ¹⁹F-unlabeled PET tracer compound (A) was able to inhibit the binding of the [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid binding in a concentration-dependent manner from CHO cells over expressing the human LPA1 receptor (TC₅₀ = 24.5 nM). In addition, two other known LPA1-selective compounds (B, C) were also able to compete with tracer in a concentration-dependent manner with IC₅₀ values of 13.9 and 35.2 nM respectively. These data demonstrate that the F-18 labelled tracer binds to LPA1.

### Example 19. In-vivo PET imaging with [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid to validate the target expression in wild type and bleomycin treated rat models

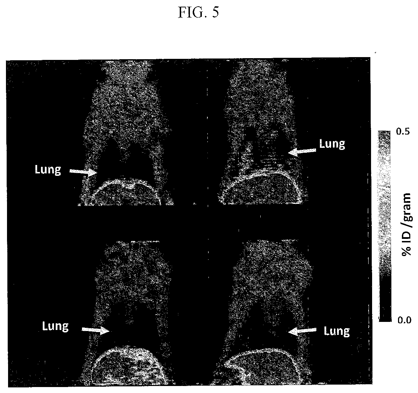

[¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid was tested to confirm its properties, specificity and targeting of the LPA1 receptor in aged-matched wild type (Sprague Dawley rat, Charles River Laboratory, PA) and bleomycin-induced pulmonary fibrosis rat models. The bleomycin-induced pulmonary fibrosis rat model was generated in anesthetized rats that were administered bleomycin (2 µL/g) via oropharyngeal instillation. For oropharyngeal instillation, anesthetized rats (isoflurane, 4% in 100% O₂) were placed on a slanted board/tilting workstation and bleomycin was dripped onto the vocal cords, facilitating aspiration. Rats were then returned to their cages until they fully recovered from anesthesia and were monitored daily for the duration of the experiment. In this experiment, the [¹⁸F]-labeled LPA1 antagonist, produced as described in the above Examples, was tested for its ability to discriminate between normal lung in wild type rats and increased lung LPA1 expression in the bleomycin rat disease model. The bleomycin treated rats were used at 14 and 15 days post bleomycin treatment. PET images were acquired at 14 days (baseline) and 15 days (blocking with a 10 mg/kg oral dose of an LPA1 antagonist administered 30 minutes before PET scan). Two groups of rats were used in the PET imaging study - aged matched wild type rats (Group 1, n=4) and bleomycin treated rats (Group 2, n=4). All animals received a baseline PET imaging scan and a repeat PET imaging scan following a single 10 mg/kg oral dose of an LPA1 antagonist. For PET image acquisition, rats were placed in an anesthetic induction chamber and 3% isoflurane inhalant anesthesia was delivered in 100% O₂ at a rate of 1-1.5 L/min. Once sedated, the rats were removed from the induction chamber and placed into a plexiglass 2-chamber holder (custom-made by BMS-Applied Biotechnology group) within the gantry of the PET system (microPET^{®} F220^{™}, Siemens Preclinical Solutions, Knoxville, TN) where they remained for the duration of the study. Anesthesia was maintained with 1-1.5% isoflurane inhalant anesthesia delivered in 100% O₂ at a rate of 2 L/min via the nose-cone. Animals were kept warm using an external standalone temperature regulating unit (M2M Imaging Corp) to prevent hypothermia during imaging. Rat respiration was continuously monitored during imaging procedures and isoflurane was adjusted dependent on depth of anesthesia. A 10-minute transmission image was first acquired using a ⁵⁷Co point source for the purpose of attenuation correction of the final PET images and confirmation of lung localization for region of interest (ROI) analysis. The scanning region was located beginning at the neck area and extending toward the hind limbs of each animal in order to place the lungs within the 7.6 cm microPET system axial field of view (FOV). Upon completion of the transmission scan, each rat received administration of between 19.5-29.7 MBq (542-803 µCi) of [¹⁸F]-(1S,3S)-3-((6-(5-((((4-(fluoro)butyl)(methyl) carbamoyl)oxy)methyl)-1-methyl-1H-1,2,3-triazol-4-yl)-2-methylpyridin-3-yl)oxy)cyclohexane-1-carboxylic acid tracer via tail vein catheter followed by a 2 hour dynamic PET emission scan. For blocking studies, the same protocol was flowed 30 minutes after a 10 mg/kg oral dose of an LPA1 antagonist. Images were reconstructed using a maximum a posteriori (MAP) algorithm with attenuation correction using the collected transmission images and corrected for radioisotope decay. The PET images were then co-registered with corresponding transmission images using anatomical landmarks (air in lung) and boundaries of the animal holder. Images were analyzed by Inveon Research Workstation (Siemens Medical Solutions USA Inc., PA). Analysis for each rat was done with regions of interest (ROIs) drawn onto each whole lung and a section of the liver using the co-registered transmission images. There were 3-4 ROIs drawn for each lung volume (left whole lung and right whole lung) as well as 3 ROIs drawn on a section of the liver. ROIs were drawn inside the whole lung to avoid spillover signal from the heart and liver. ROIs were drawn on a section of the liver using the same method. Based on quantitative values from these ROIs (nCi/cc), % injected dose/cc (%ID/g) was calculated. Radiotracer uptake was compared across lung tissues in these groups using the time periods between 60-90 minutes post radioligand injection. Using this methodology, the mean and standard error (SE) of radiotracer uptake in lung tissue at baseline was 0.080 +/- 0.007 % injected dose/gram (%ID/g) in wild type rats (Group 1) and 0.116 +/- 0.011% ID/g in bleomycin treated rats (Group 2), respectively. The lung uptake of [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in the disease induced model represents a 46% increase in the lung radioligand signal than that seen in wild type animals (p<0.05, t-Test: two-sample assuming equal variances) as shown in Table 1. The average radioligand uptake in lung tissues after treatment with an oral dose of 10 mg/kg of an LPA1 antagonist was 0.036+/- 0.003% ID/g in wild type rats (Group 1) and 0.046 +/- 0.001% ID/g in bleomycin treated rats (Group 2), respectively. When comparing the PET radioligand binding in the lung tissues of both bleomycin treated rats and wild type rats, a significant decrease (61% in bleomycin treated animals, p<0.05, and 55% in wild type animals, p<0.05, t-Test: two-sample assuming equal variances) in radioligand binding was seen in lung tissues when animals were pretreated with a 10 mg/kg oral dose of a LPA1 antagonist as shown in Table 1 and FIG. 5. These results provide in vivo evidence for specificity and targeting of LPA1 by [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid.

**Table 1: Acquisition data and lung uptake in wild type and bleomycin treated rats**

| | Body weight (gram) | Injected tracer MBq (µCi) | Lung uptake (%ID/g) @ 60-90 min |
|---|---|---|---|
| Group 1 | | | |
| WT#1 | 319 | 20.1 (542) | 0.095 |
| WT#1 + 10 mg/kg LPA1 antagonist | 319 | 25.5 (689) | 0.043 |
| WT#2 | 289 | 23.1 (526) | 0.085 |
| WT#2 + 10 mg/kg LPA1 antagonist | 289 | 28.7 (776) | 0.036 |
| WT#3 | 315 | 29.1 (785) | 0.075 |
| WT#3 + 10 mg/kg LPA1 antagonist | 315 | 25.6 (692) | 0.036 |
| WT#4 | 304 | 28.3 (764) | 0.063 |
| WT#4 + 10 mg/kg LPA1 antagonist | 304 | 29.5 (796) | 0.029 |

| Group 2 | | | |
|---|---|---|---|
| Bleo#1 | 280 | 22.5 (609) | 0.116 |
| Bleo#1 + 10 mg /kg LPA1 antagonist | 280 | 26.3 (712) | 0.047 |
| Bleo#2 | 264 | 19.5 (526) | 0.142 |
| Bleo#2 + 10 mg /kg LPA1 antagonist | 264 | 28.2 (762) | 0.045 |
| Bleo#3 | 273 | 27.0 (730) | 0.118 |
| Bleo#3 + 10 mg /kg LPA1 antagonist | 273 | 26.3 (712) | 0.044 |
| Bleo#4 | 292 | 29.7 (803) | 0.088 |
| Bleo#4 + 10 mg /kg LPA1 antagonist | 292 | 28.3 (764) | 0.047 |

FIG. 5 shows representative co-registered PET transmission and emission images summed from 60-90 minutes post injection of [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in a: A) Wild type rat B) Bleomycin treated rat. C) Wild type rat treated with 10 mg/kg of an LPA1 antagonist 30 minutes before PET scan. D) Bleomycin treated rat treated with 10 mg/kg of an LPA1 antagonist 30 minutes before PET scan.

### Example 20: In-vivo PET imaging with [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in bleomycin treated rat model to determine LPA1 target engagement of a LPA1 antagonist

[¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid was tested to validate the target engagement of an LPA1 antagonist used in a bleomycin-induced pulmonary fibrosis rat model. The generation of bleomycin-induced pulmonary fibrosis rat model, production of the [¹⁸F]-labeled LPA1 antagonist, timing of PET imaging, animal handling for PET imaging, acquisition of PET images, and post processing of PET images were as described in Example 14. In this experiment, the [¹⁸F]-labeled LPA1 antagonist was used to measure the target engagement (or % displacement) of an LPA1 antagonist at varying oral dose administrations in bleomycin treated rats. For the target engagement study, bleomycin treated rats were divided into five groups: rats in Group 1 (n=6) were pretreated with 1 mg/kg of an LPA1 antagonist; rats in Group 2 (n=6) with 3 mg/kg; rats in Group 3 (n=6) with 10 mg/kg; rats in Group 4 (n=7) with 30 mg/kg; rats in Group 5 (n=6) with 100 mg/kg. Each animal received a baseline PET scan at day 14 post bleomycin treatment. On the following day (day 15 post bleomycin treatment), animals in each group were orally dosed with an LPA1 antagonist at 30 minutes prior to [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid administration and initiation of PET scanning, as previously described. Using the methodology described in Example 14, the mean and standard error of radiotracer uptake (%ID/g) in lung tissues of bleomycin treated rats were calculated for both the baseline and on treatment (blocking) images. Measured tracer signal at baseline was 0.12 ± 0.01 %ID/g in Group 1 (1 mg/kg); 0.10 ± 0.01 %ID/g in Group 2 (3 mg/kg); 0.12 ± 0.02 %ID/g in Group 3 (10 mg/kg); 0.09 ± 0.01 %ID/g in Group 4; 0.10 ± 0.01%ID/g in Group 5. These values were compared to the %ID/g calculated for lung tissues of each rat 30 minutes after an oral dose of a LPA1 antagonist as shown in Table 2. The mean and standard error of percent displacement of [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid showed a dose dependent displacement of LPA1 antagonist: -37.9 ± 8.7% in Group 1 (1 mg/kg), -48.8 ± 11.5% in Group 2 (3 mg/kg), - 58.9 ± 7.7% in Group 3 (10 mg/kg), -66.4 ± 2.3% in Group 4 (30 mg/kg), and -56.0 ± 2.8% in Group 5 (100 mg/kg). These results provide in vivo evidence that [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid can be used to determine LPA1 target engagement and percent displacement by a LPA1 antagonist.

**Table 2 summarizes acquisition data and lung uptake of [¹⁸F]-labeled antagonist at baseline and post LPA1 antagonist treatment.**

| **LPA1 antagonist dosage** | **Rat #** | **Baseline** | | | | **After oral dose of LPA1 antagonist** | | | | **% Displacement** |
|---|---|---|---|---|---|---|---|---|---|---|
| | | **Body weight (g)** | **Injected dose MBq (µCi)** | **Tracer mass (ng/kg)** | **%ID/g in Lung** | **Body weight (g)** | **Injected dose MBq (µCi)** | **Tracer mass (ng/kg)** | **%ID/g in Lung** | |
| 1 mg/kg | Bleo1 | 288 | 20.2 (545) | 117 | 0.12 | 270 | 26.4 (713) | 176 | 0.12 | -0.6% |
| | Bleo6 | 290 | 28.2 (762) | 269 | 0.10 | 285 | 26.7 (721) | 290 | 0.08 | -26.7% |
| | Bleo13 | 262 | 30.5 (825) | 533 | 0.17 | 263 | 29.5 (796) | 375 | 0.09 | -45.8% |
| | Bleo18 | 289 | 31.0 (839) | 877 | 0.12 | 297 | 36.0 (973) | 690 | 0.06 | -46.3% |
| | Bleo21 | 391 | 34.0 (920) | 375 | 0.09 | 391 | 27.4 (740) | 162 | 0.04 | -56.9% |
| | Bleo33 | 330 | 26.9 (728) | 247 | 0.09 | 329 | 37.4 (1011) | 349 | 0.05 | -51.4% |
| | **Mean (Std.err.)** | **308 (19)** | **28.5 MBq/ 770 µci (52)** | **403 (111)** | **0.12 (0.01)** | **306 (20)** | **30.5 MBq/ 825 µci (54)** | **340 (78)** | **0.07 (0.01)** | **-37.9% (8.6%)** |
| 3 mg/kg | Bleo2 | 331 | 22.2 (600) | 112 | 0.10 | 314 | 26.6 (718) | 153 | 0.06 | -47.3% |
| | Bleo7 | 332 | 33.7 (912) | 370 | 0.10 | 335 | 37.9 (1025) | 471 | 0.10 | 5.7% |
| | Bleo14 | 310 | 27.7 (749) | 409 | 0.11 | 319 | 29.4 (794) | 309 | 0.03 | -70.4% |
| | Bleo22 | 283 | 36.8 (995) | 560 | 0.10 | 282 | 29.9 (809) | 233 | 0.04 | -63.3% |
| | Bleo31 | 372 | 25.5 (689) | 165 | 0.11 | 368 | 31.4 (848) | 193 | 0.04 | -63.8% |
| | Bleo36 | 375 | 33.2 (897) | 381 | 0.07 | 371 | 27.9 (755) | 343 | 0.03 | -57.9% |
| | **Mean (Std.err.)** | **334 (14)** | **29.9 MBq/ 807 µci (62)** | **333 (68)** | **0.10 (0.01)** | **331 (14)** | **30.5 MBq/ 825 µci (44)** | **284 (47)** | **0.05 (0.01)** | **-48.8% (11.5%)** |
| 10 mg/kg | Bleo3 | 279 | 21.6 (583) | 162 | 0.18 | 270 | **29.9** (808) | 260 | 0.15 | -21.3% |
| | Bleo8 | 312 | 34.7 (937) | 404 | 0.10 | 312 | 38.3 (1036) | 512 | 0.04 | -60.7% |
| | Bleo15 | 278 | 31.9 (863) | 709 | 0.12 | 285 | 30.0 (810) | 457 | 0.04 | -67.0% |
| | Bleo23 | 299 | 28.0 (756) | 488 | 0.09 | 303 | 29.7 (803) | 283 | 0.03 | -71.9% |
| | Bleo26 | 344 | 34.7 (939) | 696 | 0.08 | 347 | 27.6 (747) | 310 | 0.03 | -69.3% |
| | Bleo34 | 247 | 27.1 (733) | 333 | 0.13 | 251 | 39.7 (1044) | 471 | 0.05 | -63.3% |
| | **Mean (Std.err.)** | **293 (14)** | **29.7 MBq/ 802 µci (56)** | **465 (87)** | **0.12 (0.02)** | **295 (14)** | **32.3 MBq/ 874 µci (53)** | **382 (45)** | **0.05 (0.02)** | **-58.9% (7.7%)** |
| 30 mg/kg | Bleo4 | 352 | 19.4 (523) | 115 | 0.13 | 352 | 30.2 (817) | 202 | 0.05 | -57.3% |
| | Bleo11 | 334 | 32.7 (883) | 382 | 0.07 | 352 | 41.9 (1133) | 313 | 0.02 | -64.5% |
| | Bleol7 | 315 | 31.3 (846) | 811 | 0.10 | 325 | 37.9 (1025) | 665 | 0.03 | -71.4% |
| | Bleo24 | 280 | 30.0 (810) | 558 | 0.10 | 286 | 31.1 (840) | 314 | 0.02 | -75.6% |
| | Bleo27 | 327 | 29.6 (801) | 881 | 0.08 | 321 | 29.4 (794) | 474 | 0.03 | -64.0% |
| | Bleo32 | 331 | 25.9 (700) | 188 | 0.09 | 329 | 31.2 (844) | 215 | 0.03 | -63.4% |
| | Bleo37 | 375 | 33.5 (906) | 477 | 0.09 | 366 | 30.5 (825) | 464 | 0.03 | -68.6% |
| | **Mean (Std.err.)** | **331 (11)** | **28.9 MBq/ 781 µci (50)** | **487 (110)** | **0.09 (0.01)** | **333 (10)** | **33.2 MBq/ 897 µci (49)** | **378 (63)** | **0.03 (0.004)** | **-66.4% (2.3%)** |
| 100 mg/kg | Bleo5 | 331 | 27.2 (735) | 227 | 0.10 | 318 | 28.5 (770) | 278 | 0.05 | -48.4% |
| | Bleo12 | 281 | 30.4 (821) | 422 | 0.08 | 282 | 41.9 (1132) | 391 | 0.04 | -49.1% |
| | Bleo16 | 283 | 36.3 (981) | 791 | 0.15 | 299 | 29.9 (807) | 434 | 0.06 | -59.9% |
| | Bleo25 | 322 | 34.3 (927) | 735 | 0.09 | 324 | 27.9 (753) | 335 | 0.03 | -66.0% |
| | Bleo28 | 302 | 26.9 (726) | 866 | 0.11 | 304 | 28.7 (777) | 490 | 0.05 | -53.9% |
| | Bleo35 | 307 | 33.3 (901) | 468 | 0.09 | 308 | 27.8 (750) | 411 | 0.04 | -59.0% |
| | **Mean (Std.err.)** | **304 (8)** | **31.4 MBq/ 849 µci (43)** | **585 (102)** | **0.10 (0.01)** | **306 (6)** | **30.7 MBq/ 831 µci (61)** | **390 (31)** | **0.05 (0.004)** | **-56.0% (2.8%)** |

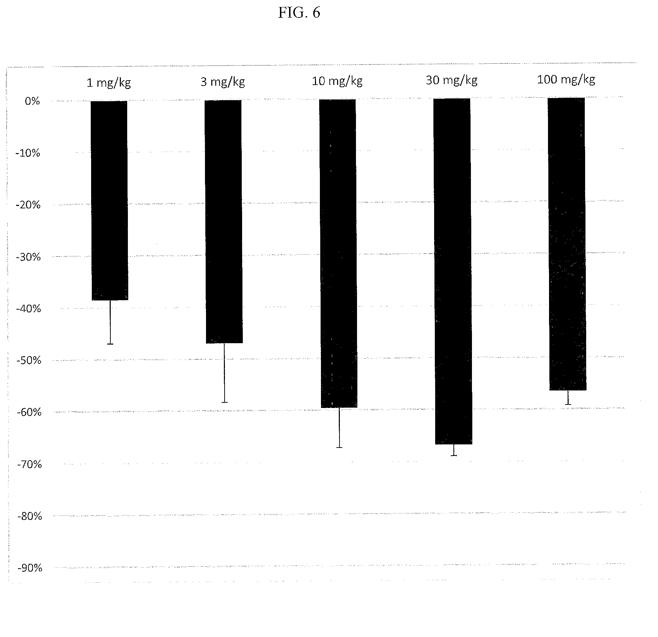

FIG. 6 shows a graphical representation of the percent displacement of [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in rat lung as function of pre-dosing multiples doses of a LPA1 antagonist. Error bars represented are standard error of each group pretreated with a LPA1 antagonist.

### Example 21. In-vivo PET imaging with [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in non-human primate to determine LPA1 target engagement of a LPA1 antagonist.

[¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid was tested as an LPA1 PET radioligand to validate the target engagement of LPA1 antagonist used in normal healthy cynomolgus monkeys and to support future clinical use of this agent as a PET radioligand. In this experiment, the specific binding to LPA1, PET imaging in normal healthy cynomolgus monkeys (n=3) was performed. PET images were acquired at baseline and again at after oral administration of an LPA1 antagonist (3, 10, and 30 mg/kg) or vehicle solution (0.5% Methocel A4M: 0.1% Tween 80 (Polysorbate 80): 99.4% Water) only. The oral dosing time of LPA1 antagonist or vehicle was 3 hours prior to [¹⁸F]-labeled antagonist injection. Cynomolgus monkeys on study were fasted beginning the morning of the imaging day. Veterinary Scientists induced initial anesthesia pre-op cocktail of 0.02mg/kg Atropine, and 5mg/kg Telazol, 0.1 mg/kg Hydromorphone IM. Under the sedation, monkeys were prepared for imaging via maintenance anesthesia with isoflurane, intubation, installation of two catheters (one catheter in saphenous for injection of [¹⁸F]-labeled antagonist and other in cephalic for saline infusion, respectively) and placement onto the animal holder for imaging. Vital signs were continuously monitored throughout the imaging study. T2-weighted Magnetic Resonance Images were first obtained in order to combine functional information from PET images with anatomical information from MRI. All MR images in this study were obtained on a 4.7-T MRI instrument (Bruker Biospin, Billerica MA) with respiratory gating prior to PET imaging. The monkey was then positioned in a ventral decubitus position and routine MRI-compatible monitors were attached. The animal was wrapped in blankets and positioned within a quadrature 21 cm coil for radiofrequency transmitting and receiving. Following initial scout images, high resolution coronal images were acquired using the Bruker RARE sequence with the following parameters: TR/TE=3100/40 ms, FOV=18 cm², Matrix=256², slice thickness = 5 mm, 33 axial slices were acquired, with four signal averages and an acquisition time of 15 minutes to cover from the brain to the lung area. At the completion of MRI imaging, the anesthetized monkeys in the same extended imaging bed were immediately transported from the MRI system to the microPET system (microPET^{®} F220^{™}, Siemens Preclinical Solutions, Knoxville, TN). A 10 minute transmission scan of the lung region was first acquired using a ⁵⁷Co point source for the purpose of attenuation correction of the final PET images and confirmation of lung localization for region of interest (ROI) analysis. Upon completion of the transmission scan, each monkey was then injected via saphenous vein with 100.3-59.2 MBq ( 2.7-1.6 mCi) of [¹⁸F]-(1S,3S)-3-((6-(5-((((4-(fluoro)butyl)(methyl)carbamoyl)oxy) methyl)-1-methyl-1H-1,2,3-triazol-4-yl)-2-methylpyridin-3-yl)oxy)cyclohexane-1-carboxylic acid tracer followed by acquisition of a 2 hour dynamic PET emission scan. For on treatment ( blocking studies) imaging, the same protocol was followed 3 hours after oral dose of the LPA1 antagonist or vehicle as described earlier. After imaging was completed, the monkey was moved out of the holder for recovery on a blanketed heating pad and administered supplemental oxygen. After returning to a conscious state, the monkey was transferred to an isolation room with food and water until the radioactivity was decayed to background levels.

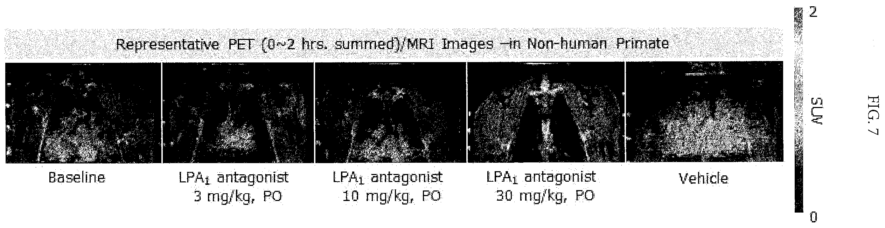

PET images were reconstructed with a maximum a posteriori (MAP) algorithm with attenuation correction using the collected transmission images and corrected for radioisotope decay. Using the AMIDE software system (version 0.9.1, amide.sourceforge.net), PET images were manually co-registered with corresponding MRI images guided by fiducial and anatomical landmarks. ROIs in both lungs were manually drawn in the axial co-registered PET/MRI. Mean standardized uptake value (SUV),which is normalized by animal body weight (kg) and injected radioative dose (mCi), was calculated. At baseline, average SUV for individual animals was 0.398 (animal B6203), 0.321 (animal B7111), and 0.391 (animal B7112). For calculating the % displacement from baseline study, average SUV from 60-90 min following tracer administration was used and compared to the baseline values for each individual animal. Table 3 shows acquisition data, LPA1 antagonist exposure in plasma at 3 hr post dosing, and average SUV from 60-90 min in each monkey for each imaging scan. Figure 7 shows representative PET images at baseline and following administration of vehicle or LPA1 antagonist at 3, 10, and 30 mg/kg. The percent displacement (mean ± standard error) calculated for each treatment group compared to baseline was as follows: 7.7 ± 1.1% (vehicle), -30.8 ± 12.0% (3 mg/kg LPA1 antagonist), -53.7 ± 3.7% (10 mg/kg LPA1 antagonist), and -60.3 ± 7.4% (30 mg/kg LPA1 antagonist) (Figure 6). These results demonstrate that [¹⁸F]-(1S,3S)-3-((6-(5-((((4-(fluoro)butyl)(methyl)carbamoyl)oxy) methyl)-1-methyl-1H-1,2,3-triazol-4-yl)-2-methylpyridin-3-yl)oxy)cyclohexane-1-carboxylic acid may be a viable PET imaging radioligand for assessment LPA1 expression and target engagement.

**Table 3 summarizes PET Acquisition data and lung uptake (average SUV from 60-90 min) in healthy cynomolgus monkeys with [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid at baseline and with pre-treatment of an LPA1 antagonist.**

| **Group** | **Body weight (kg)** | **Injected dose MBq (µCi)** | **Tracer mass (µg/kg)** | **SUV in Lung (60-90 min)** | **LPA1 antagonist plasma exposure at 3 hr post dose (nM)** |
|---|---|---|---|---|---|
| ***Monkey 1 (Male)*** | | | | | |
| 1^{st} Baseline | 4.5 | 72.7 (1964) | 0.056 | 0.459 | 0 |
| 2^{nd} Baseline | 4.6 | 80.2 (2168) | 0.053 | 0.307 | 0 |
| 3^{rd} Baseline | 4.5 | 86.9 (2349) | 0.056 | 0.428 | 0 |
| LPA1 antagonist 3 mg/kg | 4.5 | 71.4 (1930) | 0.043 | 0.184 | 729 |
| LPA1 antagonist 10 mg/kg | 4.5 | 67.9 (1836) | 0.053 | 0.191 | 5008 |
| LPA1 antagonist 30 mg/kg | 4.4 | 63.9 (1727) | 0.041 | 0.102 | 13202 |
| Vehicle | 4.7 | 100.3 (2711) | 0.085 | 0.433 | 0 |

| ***Monkey 2 (Male)*** | | | | | |
|---|---|---|---|---|---|
| 1^{st} Baseline | 3.8 | 59.2 (1599) | 0.077 | 0.333 | 0 |
| 2^{nd} Baseline | 3.9 | 75.9 (2051) | 0.067 | 0.273 | 0 |
| 3^{rd} Baseline | 4.1 | 81.2 (2194) | 0.096 | 0.356 | 0 |
| LPA1 antagonist 3 mg/kg | 4.0 | 64.7 (1749) | 0.058 | 0.238 | 743 |
| LPA1 antagonist 10 mg/kg | 3.9 | 89.4 (2417) | 0.080 | 0.165 | 3983 |
| LPA1 antagonist 30 mg/kg | 3.7 | 75.6 (2042) | 0.087 | 0.137 | 16848 |
| Vehicle | 4.2 | 82.8 (2239) | 0.116 | 0.342 | 0 |

| ***Monkey 3 (Male)*** | | | | | |
|---|---|---|---|---|---|
| 1^{st} Baseline | 4.1 | 94.8 (2561) | 0.068 | 0.379 | 0 |
| 2^{nd} Baseline | 4.1 | 78.9 (2133) | 0.065 | 0.403 | 0 |
| 3^{rd} Baseline | NA | NA | NA | NA | NA |
| LPA1 antagonist 3 mg/kg | 4.2 | 71.9 (1942) | 0.047 | 0.341 | 356 |
| LPA1 antagonist 10 mg/kg | 4.0 | 78.0 (2108) | 0.055 | 0.153 | 2435 |
| LPA1 antagonist 30 mg/kg | 3.8 | 75.1 (2029) | 0.080 | 0.198 | 7530 |
| Vehicle | NA | NA | NA | NA | NA |

FIG. 7 shows representative PET/MRI Images of [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in cynomolgus monkey at baseline and after administration of vehicle or LPA1 antagonist at 3, 10, and 30 mg/kg.

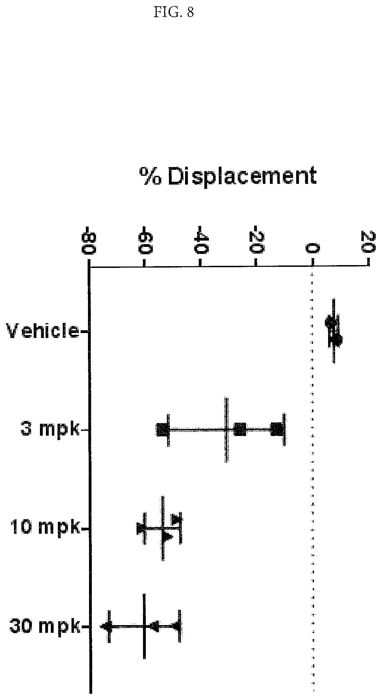

FIG. 8 shows a graphical representation of the percent displacement of [¹⁸F]-(1S,3S)-3-((6-(5-((((4-(fluoro)butyl)(methyl)carbamoyl)oxy)methyl)-1-methyl-1H-1,2,3-triazol-4-yl)-2-methylpyridin-3-yl)oxy)cyclohexane-1-carboxylic acid in cynomolgus monkey lung tissues after treatment with a LPA1 antagonist or vehicle. Error bars are represented as standard error for each group.

It was discovered that the compound of the present invention offers several advantages and design features that make it a more useful PET radioligand to determine LPA1 target engagement and dose dependent receptor occupancy. For example, 1) the PET radioligand of the present invention has free fractions that are within the 5-11 % free of protein binding, leading to more radioligand signal within the lung tissues for quantification; 2) its radio-metabolism is 80% intact at the 90 minute post-injection with more radioligand signal within the lung tissues for quantification of the LPA1 receptor; 3) it has improved liver to lung ratios (the liver to lung ratios to 8:1 in the wild type rat, 5:1 in bleomycin treated rats and 7:1 in the non-human primate) which lead to a signal within the lung tissues that is more quantifiable; and 4) it has an increased isolated radiochemical yield ( 370 mCi vs 25 mCi), is labeled with fluorine-18, which has a 110 minute half-life compared to carbon-11 which has a 20 minute half-life, has a higher effective specific activity ( 5.0 mCi/nmol vs. 2.2 mCi/nmol). All of these factors combine leads to a signal within the lung tissues that is more quantifiable.

## Claims

1. A radiolabeled compound of Formula (I): 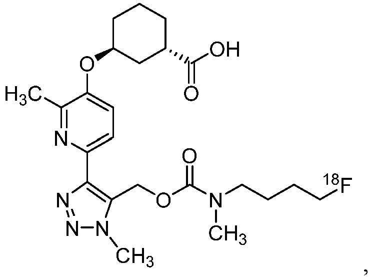 or a pharmaceutically acceptable salt thereof.

2. A pharmaceutical composition comprising the radiolabeled compound of claim 1, or a pharmaceutically acceptable salt thereof; and a pharmaceutically acceptable carrier therefor.

3. A method of *in vivo* imaging of mammalian tissues of known LPA1 expression comprising the steps of:
(a) administering the radiolabeled compound of claim 1, or a pharmaceutically acceptable salt thereof, to a mammalian species; and
(b) imaging *in vivo* the distribution of the radiolabeled compound by positron emission tomography (PET) scanning.

4. A method for screening a non-radiolabeled compound to determine its affinity for occupying the binding sites of LPA1 receptors in mammalian tissue comprising the steps of:
(a) administering the radiolabeled compound of claim 1, or a pharmaceutically acceptable salt thereof, to a mammalian species;
(b) imaging *in vivo* tissues of known LPA1 expression by positron emission tomography (PET) to determine a baseline uptake of the radiolabeled compound;
(c) administering the non-radiolabeled compound, or a pharmaceutically acceptable salt thereof, to the mammalian species;
(d) administering a second dose of the radiolabeled compound to the mammalian species;
(e) imaging *in vivo* the distribution of the radiolabeled compound in tissues that express LPA1 receptors;
(f) comparing the signal from PET scan data at the baseline within the tissue that expresses LPA1 to PET scan data retrieved after administering the non-radiolabeled compound within the tissue that expresses LPA1 receptors.

5. A method for monitoring the treatment of a mammalian patient who is being treated with an LPA1 receptor antagonist comprising the steps of:
(a) administering to the patient the radiolabeled compound of claim 1, or a pharmaceutically acceptable salt thereof;
(b) obtaining an image of tissues in the patient that express LPA1 receptors by positron emission tomography (PET); and
(c) detecting to what degree the radiolabeled compound occupies the binding site of the LPA1 receptor.

6. A method for tissue imaging comprising the steps of
contacting a tissue that contains LPA1 receptors with the radiolabeled compound of claim 1, or a pharmaceutically acceptable salt thereof; and
detecting the radiolabeled compound using positron emission tomography (PET) imaging.

7. The method of claim 6 wherein the radiolabeled compound is detected *in vitro.*

8. The method of claim 6 wherein the radiolabeled compound is detected *in vivo.*

9. The radiolabeled compound of claim 1, or a pharmaceutically acceptable salt thereof, for use in a method for diagnosing the presence of a fibrotic disease in a mammalian species, the method comprising the steps of:
(a) administering to a mammalian species in need thereof the radiolabeled compound of claim 1, or a pharmaceutically acceptable salt thereof, which binds to the LPA1 receptor associated with the presence of the fibrotic disease; and
(b) obtaining a radio-image of at least a portion of the mammalian species to detect the presence or absence of the radiolabeled compound; wherein the presence and location of the radiolabeled compound above background is indicative of the presence or absence of the fibrotic disease.

10. The radiolabeled compound of claim 1, or a pharmaceutically acceptable salt thereof, for use in a method for diagnosing the presence of a fibrotic disease in a mammalian species, the method comprising the steps of:
(a) administering to a mammalian species in need thereof the radiolabeled compound of claim 1, or a pharmaceutically acceptable salt thereof, which binds to the LPA1 receptor associated with the presence of the fibrotic disease;
(b) detecting radioactive emission of the radiolabeled compound for the mammalian species;
(c) comparing the radioactive emission from the radiolabeled compound for the mammalian species with standard values; and
(d) finding any significant deviation between the radioactive emission detected for the mammalian species as compared with standard values, and attributing the deviation to the fibrotic disease.

11. The compound for use of claim 9 or claim 10, wherein the fibrotic disease is idiopathic pulmonary fibrosis.

12. A method for quantifying diseased cells or tissues in a mammalian species, comprising the steps of
(a) administering to a mammalian species having diseased cells or tissues the radiolabeled compound of claim 1, or a pharmaceutically acceptable salt thereof, which binds to LPA1 receptors located within the diseased cells or tissues; and
(b) detecting radioactive emissions of the radiolabeled compound in the diseased cells or tissues, wherein the level and distribution of the radioactive emissions in the diseased cells or tissues are a quantitative measure of the diseased cells or tissues.

## Patentansprüche

1. Radioaktiv markierte Verbindung mit der Formel (I): 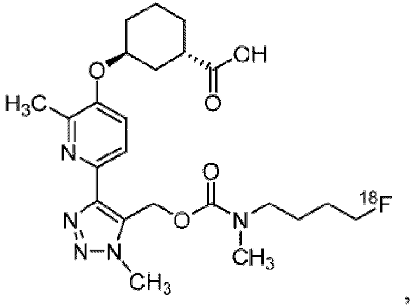 oder pharmazeutisch unbedenkliches Salz davon.

2. Pharmazeutische Zusammensetzung, umfassend die radioaktiv markierte Verbindung nach Anspruch 1 oder ein pharmazeutisch unbedenkliches Salz davon und einen pharmazeutisch unbedenklichen Trägerstoff dafür.

3. Verfahren zum Abbilden von Säugetiergeweben mit bekannter LPA1-Expression *in vivo,* umfassend die Schritte:
(a) Verabreichen der radioaktiv markierten Verbindung nach Anspruch 1 oder eines pharmazeutisch unbedenklichen Salzes davon an eine Säugetierspezies und
(b) Abbilden der Verteilung der radioaktiv markierten Verbindung *in vivo* durch Positron-Emissionstomographie-Scannen (PET-Scannen).

4. Verfahren zum Screenen einer nicht radioaktiv markierten Verbindung, um ihre Affinität für ein Belegen der Bindungsstellen von LPA1-Rezeptoren in Säugetiergewebe zu bestimmen, umfassend die Schritte:
(a) Verabreichen der radioaktiv markierten Verbindung nach Anspruch 1 oder eines pharmazeutisch unbedenklichen Salzes davon an eine Säugetierspezies;
(b) Abbilden von Geweben mit bekannter LPA1-Expression *in vivo* durch Positron-Emissionstomographie (PET), um eine Baseline-Aufnahme der radioaktiv markierten Verbindung zu bestimmen;
(c) Verabreichen der nicht radioaktiv markierten Verbindung oder eines pharmazeutisch unbedenklichen Salzes davon an die Säugetierspezies;
(d) Verabreichen einer zweiten Dosis der radioaktiv markierten Verbindung an die Säugetierspezies;
(e) Abbilden der Verteilung der radioaktiv markierten Verbindung in Geweben, die LPA1-Rezeptoren exprimieren, *in vivo;*
(f) Vergleichen des Signals von PET-Scandaten zur Baseline innerhalb des Gewebes, das LPA1 exprimiert, mit PET-Scandaten, die nach Verabreichen der nicht radioaktiv markierten Verbindung innerhalb des Gewebes, das LPA1-Rezeptoren exprimiert, abgerufen werden.

5. Verfahren zum Überwachen der Behandlung eines Säugetierpatienten, der mit einem LPA1-Rezeptor-Antagonisten behandelt wird, umfassend die Schritte:
(a) Verabreichen der radioaktiv markierten Verbindung nach Anspruch 1 oder eines pharmazeutisch unbedenklichen Salzes davon an den Patienten;
(b) Beziehen eines Bilds von Geweben in dem Patienten, die LPA1-Rezeptoren exprimieren, durch Positron-Emissionstomographie (PET) und
(c) Erfassen, in welchem Ausmaß die radioaktiv markierte Verbindung die Bindungsstelle des LPA1-Rezeptors belegt.

6. Verfahren zum Gewebeabbilden, umfassend die folgenden Schritte:
Inkontaktbringen eines Gewebes, das LPA1-Rezeptoren enthält, mit der radioaktiv markierten Verbindung nach Anspruch 1 oder einem pharmazeutisch unbedenklichen Salz davon und
Erfassen der radioaktiv markierten Verbindung unter Verwendung von Positron-Emissionstomographie-Abbildung (PET-Abbildung).

7. Verfahren nach Anspruch 6, wobei die radioaktiv markierte Verbindung *in vitro* erfasst wird.

8. Verfahren nach Anspruch 6, wobei die radioaktiv markierte Verbindung *in vivo* erfasst wird.

9. Radioaktiv markierte Verbindung nach Anspruch 1 oder pharmazeutisch unbedenkliches Salz davon zur Verwendung in einem Verfahren zum Diagnostizieren des Vorliegens einer fibrotischen Erkrankung bei einer Säugetierspezies, wobei das Verfahren die Schritte umfasst:
(a) Verabreichen der radioaktiv markierten Verbindung nach Anspruch 1 oder eines pharmazeutisch unbedenklichen Salzes davon, die bzw. das an den LPA1-Rezeptor, der mit dem Vorliegen der fibrotischen Erkrankung assoziiert ist, bindet, an eine Säugetierspezies, die dieser bedarf; und
(b) Erlangen eines Radiobilds von mindestens einem Teil der Säugetierspezies, um das Vorliegen oder die Abwesenheit der radioaktiv markierten Verbindung zu erfassen; wobei das Vorliegen und der Ort der radioaktiv markierten Verbindung über einem Hintergrund das Vorliegen oder die Abwesenheit der fibrotischen Erkrankung angeben.

10. Radioaktiv markierte Verbindung nach Anspruch 1 oder pharmazeutisch unbedenkliches Salz davon zur Verwendung in einem Verfahren zum Diagnostizieren des Vorliegens einer fibrotischen Erkrankung bei einer Säugetierspezies, wobei das Verfahren die folgenden Schritte umfasst:
(a) Verabreichen der radioaktiv markierten Verbindung nach Anspruch 1 oder eines pharmazeutisch unbedenklichen Salzes davon, die bzw. das an den LPA1-Rezeptor, der mit dem Vorliegen der fibrotischen Erkrankung assoziiert ist, bindet, an eine Säugetierspezies, die dieser bedarf;
(b) Erfassen einer radioaktiven Emission der radioaktiv markierten Verbindung für die Säugetierspezies;
(c) Vergleichen der radioaktiven Emission von der radioaktiv markierten Verbindung für die Säugetierspezies mit Standardwerten und
(d) Finden jeglicher signifikanten Abweichung zwischen der für die Säugetierspezies erfassten radioaktiven Emission im Vergleich zu Standardwerten und Zuordnen der Abweichung zu der fibrotischen Erkrankung.

11. Verbindung zur Verwendung nach Anspruch 9 oder 10, wobei die fibrotische Erkrankung eine idiopathische Lungenfibrose ist.

12. Verfahren zum Quantifizieren erkrankter Zellen oder Gewebe bei einer Säugetierspezies, umfassend die folgenden Schritte:
(a) Verabreichen der radioaktiv markierten Verbindung nach Anspruch 1 oder eines pharmazeutisch unbedenklichen Salzes davon, die bzw. das an LPA1-Rezeptoren, die sich innerhalb von erkrankten Zellen oder Geweben befinden, bindet, an eine Säugetierspezies mit erkrankten Zellen oder Geweben und
(b) Erfassen von radioaktiven Emissionen der radioaktiv markierten Verbindung in den erkrankten Zellen oder Geweben, wobei das Niveau und die Verteilung der radioaktiven Emissionen in den erkrankten Zellen oder Geweben ein quantitatives Maß der erkrankten Zellen oder Gewebe ist.

## Revendications

1. Composé radiomarqué répondant à la formule (I) : 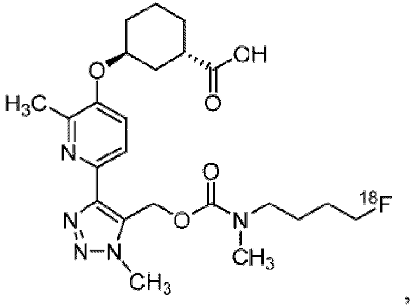 ou sel pharmaceutiquement acceptable de celui-ci.

2. Composition pharmaceutique comprenant le composé radiomarqué selon la revendication 1 ou un sel pharmaceutiquement acceptable de celui-ci et un support pharmaceutiquement acceptable pour celui-ci.

3. Procédé d'imagerie *in vivo* de tissus de mammifères présentant une expression connue de LPA1, comprenant les étapes suivantes :
(a) administration du composé radiomarqué selon la revendication 1 ou d'un sel pharmaceutiquement acceptable de celui-ci à une espèce de mammifère ; et
(b) imagerie *in vivo* de la répartition du composé radiomarqué par tomographie par émission de positrons (TEP).

4. Procédé de criblage d'un composé non radiomarqué pour déterminer son affinité à occuper les sites de liaison des récepteurs LPA1 dans des tissus de mammifères, comprenant les étapes suivantes :
(a) administration du composé radiomarqué selon la revendication 1 ou d'un sel pharmaceutiquement acceptable de celui-ci à une espèce de mammifère ;
(b) imagerie *in vivo* de tissus présentant une expression connue de LPA1 par tomographie par émission de positrons (TEP) pour déterminer une absorption de référence du composé radiomarqué ;
(c) administration du composé non radiomarqué ou d'un sel pharmaceutiquement acceptable de celui-ci à l'espèce mammifère ;
(d) administration d'une deuxième dose du composé radiomarqué à l'espèce mammifère ;
(e) imagerie *in vivo* de la répartition du composé radiomarqué dans des tissus qui expriment les récepteurs LPA1 ;
(f) comparaison du signal issu de données de balayage de TEP au moment de référence au sein du tissu qui exprime LPA1 à des données de balayage de TEP relevées après l'administration du composé non radiomarqué au sein du tissu qui exprime les récepteurs LPA1.

5. Procédé de surveillance du traitement d'un patient mammifère traité par un antagoniste des récepteurs LPA1, comprenant les étapes suivantes :
(a) administration, au patient, du composé radiomarqué selon la revendication 1 ou d'un sel pharmaceutiquement acceptable de celui-ci ;
(b) obtention d'une image de tissus du patient qui expriment les récepteurs LPA1 par tomographie par émission de positrons (TEP) ; et
(c) détection de la mesure dans laquelle le composé radiomarqué occupe le site de liaison du récepteur LPA1.

6. Procédé d'imagerie tissulaire comprenant les étapes suivantes :
mise en contact d'un tissu contenant des récepteurs LPA1 avec le composé radiomarqué selon la revendication 1 ou un sel pharmaceutiquement acceptable de celui-ci ; et
détection du composé radiomarqué par imagerie par tomographie par émission de positrons (TEP).

7. Procédé selon la revendication 6, dans lequel le composé radiomarqué est détecté *in vitro.*

8. Procédé selon la revendication 6, dans lequel le composé radiomarqué est détecté *in vivo.*

9. Composé radiomarqué selon la revendication 1, ou sel pharmaceutiquement acceptable de celui-ci, pour utilisation dans une méthode de diagnostic de la présence d'une maladie fibrotique chez une espèce de mammifère, la méthode comprenant les étapes suivantes :
(a) administration, à une espèce de mammifère en ayant besoin, du composé radiomarqué selon la revendication 1, ou d'un sel pharmaceutiquement acceptable de celui-ci, qui se lie au récepteur LPA1 associé à la présence de la maladie fibrotique ; et
(b) obtention d'une image radiologique d'au moins une partie de l'espèce de mammifère pour détecter la présence ou l'absence du composé radiomarqué ; la présence et l'emplacement du composé radiomarqué au-dessus d'un bruit de fond étant indicatifs de la présence ou de l'absence de la maladie fibrotique.

10. Composé radiomarqué selon la revendication 1, ou sel pharmaceutiquement acceptable de celui-ci, pour utilisation dans une méthode de diagnostic de la présence d'une maladie fibrotique chez une espèce de mammifère, la méthode comprenant les étapes suivantes :
(a) administration, à une espèce de mammifère en ayant besoin, du composé radiomarqué selon la revendication 1, ou d'un sel pharmaceutiquement acceptable de celui-ci, qui se lie au récepteur LPA1 associé à la présence de la maladie fibrotique ;
(b) détection d'une émission radioactive du composé radiomarqué pour l'espèce mammifère ;
(c) comparaison de l'émission radioactive issue du composé radiomarqué pour l'espèce mammifère à des valeurs standard ; et
(d) repérage de tout écart significatif entre l'émission radioactive détectée pour l'espèce mammifère et lesdites valeurs standard, et attribution dudit écart à la maladie fibrotique.

11. Composé pour utilisation selon la revendication 9 ou la revendication 10, ladite maladie fibrotique étant la fibrose pulmonaire idiopathique.

12. Procédé de quantification de cellules ou de tissus malades chez une espèce de mammifère, comprenant les étapes suivantes :
(a) administration, à une espèce de mammifère ayant des cellules ou des tissus malades, du composé radiomarqué selon la revendication 1, ou d'un sel pharmaceutiquement acceptable de celui-ci, qui se lie aux récepteurs LPA1 situés dans les cellules ou les tissus malades ; et
(b) détection d'émissions radioactives du composé radiomarqué dans les cellules ou tissus malades, le niveau et la répartition des émissions radioactives dans les cellules ou tissus malades étant une mesure quantitative des cellules ou tissus malades.
